# NEUE EUROPÄISCHE PATENTSCHRIFT

(11) **EP 0 430 863 B2**
(45) Veröffentlichungstag und Bekanntmachung des Hinweises auf die Entscheidung über den Einspruch: **25.08.1999**
(45) Hinweis auf die Patenterteilung: 17.05.1995
(21) Anmeldenummer: 90730017.2
(22) Anmeldetag: 19.11.1990
(51) Int. Cl.: C08G 69/48, C08G 73/02, A61K 49/00

(54) **Kaskadenpolymer-gebundene Komplexbildner, deren Komplexe und Konjugate, Verfahren zu ihrer Herstellung und diese enthaltende pharmazeutische Mittel**
Complexe builders bonded to sterwise prepared polymers, their complexes and conjugates, process for their preparation and pharmaceutical agents containing the same
Formateur de complexe lié à un polymère préparé par étapes, ses complexes et conjugats, procédé de préparation et substances pharmaceutiques les contenant

(30) Priorität: 21.11.1989 DE 3938992
(43) Veröffentlichungstag der Anmeldung: 05.06.1991
(73) Patentinhaber: SCHERING AKTIENGESELLSCHAFT, 13342 Berlin (DE)
(72) Erfinder: Platzek, Johannes, Dr., W-1000 Berlin 33 (DE); Schmitt-Willich, Heribert, Dr., W-1000 Berlin 45 (DE); Gries, Heinz, Dr., W-1000 Berlin 31 (DE); Schuhmann-Giampieri, Gabriele, Dr., W-1000 Berlin 45 (DE); Vogler, Hubert, Dr., W-1000 Berlin 12 (DE); Weinmann, Hanns-Joachim, Dr., W-1000 Berlin 38 (DE); Bauer, Hans, Dr., W-1000 Berlin 42 (DE)
(74) Vertreter: Lederer, Franz, Dr.

(56) Entgegenhaltungen:
- EP-A- 0 169 299
- EP-A- 0 232 751
- EP-A- 0 271 180
- EP-A- 0 277 088
- EP-A- 0 292 689
- EP-A- 0 305 320
- EP-A- 0 331 616
- EP-A- 0 481 526
- AU-A- 634 167
- FR-A- 2 539 996
- FR-A- 2 590 484
- US-A- 647 447
- US-A- 4 587 329
- US-A- 4 957 939
- US-A- 4 963 344
- BIOCHEMICA ET BIOPHYSICA ACTA 883 (1986), Amsterdam; Y. MANABE e.a. "High- Level Conjugation of Chelating Agents onto Immunoglobulins: use of an intermediary" & Poly(L Lysine)- Diethylenetriaminepenta - Acetic Acid Carrier
- Polymer Journal, Tomalia et al, 17, 117 (1985)
- Chemistry in Britain, vol. 30, nr. 8, 1994
- European Magnetic Resonance Forum: "New Developments in Contrast Agent Research", Proceedings of the 2nd Special Topic Seminar, 26-28 Sept. 1990 (Bordeaux), pages 75 and 78

## Beschreibung

Die Erfindung betrifft die in den Patentansprüchen gekennzeichneten Gegenstände, das heißt neue Kaskaden-Polymer-Komplexe, diese Verbindungen enthaltende Mittel, die Verwendung der Komplexe in der Diagnostik sowie Verfahren zur Herstellung dieser Mittel.

Magnevist (GdDTPA/dimeglumin) ist das erste registrierte Kontrastmittel für die Kernspintomographie (MRI = Magnetic Resonance Imaging). Es ist besonders gut für die Diagnose pathologischer Bereiche (z. B. Entzündungen, Tumore etc.) geeignet. Die Verbindung wird nach intravenöser Injektion über die Nieren eliminiert; eine extrarenale Ausscheidung wird praktisch nicht beobachtet.

Ein Nachteil von Magnevist ist, daß es sich nach intravenöser Applikation gleichmäßig Zwischen dem vasalen und dem interstitiellen Raum verteilt. Somit ist eine Abgrenzung der Gefäße gegenüber dem umliegenden interstitiellen Raum bei Anwendung von Magnevist nicht möglich.

Besonders für die Darstellung von Gefäßen wäre ein Kontrastmittel wünschenswert, das sich ausschließlich im vasalen Raum (Gefäßraum) verteilt. Ein solches blood-pool-agent soll es ermöglichen, mit Hilfe der Kernspintomographie gut durchblutetes von schlecht durchblutetem Gewebe abzugrenzen und somit eine Ischämie zu diagnostizieren. Auch infarziertes Gewebe ließe sich aufgrund seiner Anämie vom umliegenden gesunden oder ischamischen Gewebe abgrenzen, wenn ein vasales Kontrastmittel angewandt wird. Dies ist von besonderer Bedeutung, wenn es z. B. darum geht, einen Herzinfarkt von einer Ischämie zu unterscheiden.

Bisher müssen sich die meisten der Patienten, bei denen Verdacht auf eine kardiovaskuläre Erkrankung besteht (diese Erkrankung ist die häufigste Todesursache in den westlichen Industrieländern), invasiven diagnostischen Untersuchungen unterziehen. In der Angiographie wird zur Zeit vor allem die RontgenDiagnostik mit Hilfe von jodhaltigen Kontrastmitteln angewandt. Diese Untersuchungen sind mit verschiedenen Nachteilen behaftet: sie sind mit dem Risiko der Strahlenbelastung verbunden, sowie mit Unannehmlichkeiten und Belastungen, die vor allem daher kommen, daß die jodhaltigen Kontrastmittel, verglichen mit NMR-Kontrastmitteln, in sehr viel höherer Konzentration angewandt werden müssen.

Es besteht daher ein Bedarf an NMR-Kontrastmitteln, die den vasalen Raum markieren können (blood-pool-agent). Diese Verbindungen sollen sich durch eine gute Verträglichkeit und durch eine hohe Wirksamkeit (hohe Steigerung der Signalintensität beim MRI) auszeichnen.

Der Ansatz, zumindest einen Teil dieser Probleme durch Verwendung von Komplexbildnern, die an Makro- oder Biomoleküle gebunden sind, zu lösen, war bisher nur sehr begrenzt erfolgreich.

So ist beispielsweise die Anzahl paramagnetischer Zentren in den Komplexen, die in den Europäischen Patentanmeldungen Nr. 88 695 und Nr. 150 884 beschrieben sind, für eine zufriedenstellende Bildgebung nicht ausreichend.

Erhöht man die Anzahl der benötigten Metallionen durch mehrfache Einführung komplexierender Einheiten in ein Makromolekül, so ist das mit einer nicht tolerierbaren Beeinträchtigung der Affinität und/oder Spezifizität dieses Makromoleküls verbunden [J. Nucl. Med. 24, 1158 (1983)].

Makromoleküle sind generell als Kontrastmittel für die Angiographie geeignet, Albumin-GdDTPA (Radiology 1987; 162: 205) z.B. zeigt jedoch 24 Stunden nach intravenöser Injektion bei der Ratte eine Anreicherung im Lebergewebe, die fast 30 % der Dosis ausmacht. Außerdem werden in 24 Stunden nur 20 % der Dosis eliminiert.

Das Makromolekül Polylysin-GdDTPA (Europäische Patentanmeldung, Publikations-Nr. 0 233 619) erwies sich ebenfalls geeignet als blood-pool-agent. Diese Verbindung besteht jedoch herstellungsbedingt aus einem Gemisch von Molekülen verschiedener Große. Bei Ausscheidungsversuchen bei der Ratte konnte gezeigt werden, daß dieses Makromolekül unverändert durch glomeruläre Filtration über die Niere ausgeschieden wird. Synthesebedingt kann Polylysin-GdDTPA aber auch Makromoleküle enthalten, die so groß sind, daß sie bei der glomerulären Filtration die Kapillaren der Niere nicht passieren können und somit im Körper zurückbleiben.

Auch makromolekulare Kontrasmittel auf der Basis von Kohlenhydraten, z.B. Dextran, sind beschrieben worden (Europäische Patentanmeldung, Publikations-Nr. 0 326 226). Der Nachteil dieser Verbindungen liegt darin, daß diese in der Regel nur 4.6 % des signalverstärkenden paramagnetischen Kations tragen.

Es bestand daher die Aufgabe, neue diagnostische Mittel vor allem zur Erkennung und Lokalisierung von Gefäßkrankheiten, die die genannten Nachteile nicht besitzen, zur Verfügung zu stellen. Diese Aufgabe wird durch die vorliegende Erfindung gelöst.

Es wurde gefunden, daß sich Kaskaden-Polymer Komplexe, mit komplexbildenden Liganden überraschenderweise hervorragend zur Herstellung von NMR- und Röntgen-Diagnostika eignen, die die genannten Nachteile nicht aufweisen.

Gegenstand der Erfindung sind Kaskaden-Polymer Komplexe der allgemeinen Formel I, die komplexbildende Liganden enthalten, 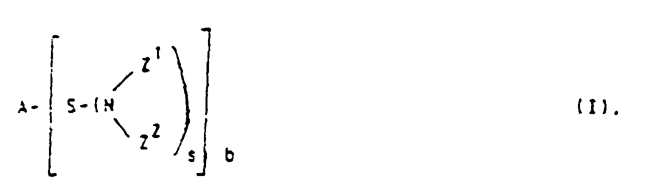 worin
- A: für einen Stickstoff-haltigen Kaskadenkern der Basismultiplizität b,
- S: für eine Reproduktionseinheit,
- N: für ein Stickstoffatom,
- Z¹ und Z²: für die erste bis vorletzte Generation jeweils für 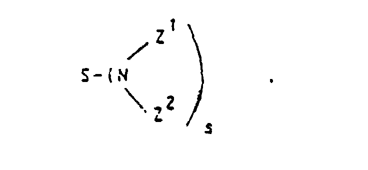 für die letzte Generation dagegen
- Z¹: für ein Wasserstoffatom, einen gegebenenfalls 1 bis 3 Carboxyl-, 1 bis 3 Sulfonsäure-, 1 bis 5 Hydroxygruppen und/oder 1 bis 3 Sauerstoffatome enthaltenden C₁-C₁₀-Alkyl-, C₂-C₁₀-Acyl- oder C₁-C₁₀-Alkylsulfonylrest oder für den Rest eines Komplexes K und
- Z²: für den Rest eines Komplexes K
- b: für die Ziffern 1 bis 50 und
- s: für die Ziffern 1 bis 3 stehen,
- K: einen an die terminalen Stickstoffatome der letzten Generation über V gebundenen Komplex-Rest der allgemeinen Formel (IB) bedeutet 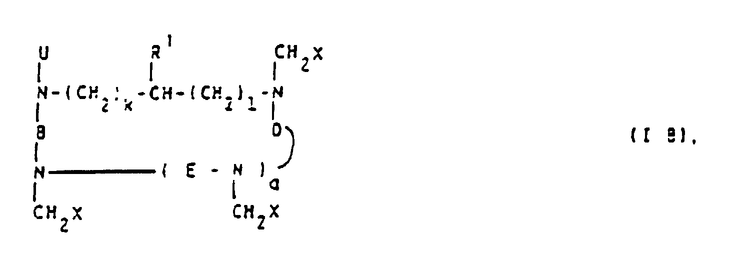 worin
k für die Ziffern 1,2,3,4 oder 5,
l für die Ziffern 0,1,2,3,4 oder 5,
q für die Ziffern 0,1 oder 2,
U für CH₂X oder V
X für den Rest -COOH stehen,
B,D und E die gleich oder verschieden sind, jeweils für die Gruppe -(CH₂)ₐ- mit a in der Bedeutung der Ziffern 2,3,4 oder 5,
R¹ für V oder ein Wasserstoffatom stehen,
mit der Maßgabe, daß R¹ nur dann für V steht, wenn U gleichzeitig CH₂X bedeutet, und daß U nur dann für V steht, wenn gleichzeitig R¹ ein Wasserstoffatom bedeutet, sowie der Maßgabe, daß gewünschtenfalls ein Teil der COOH-Gruppen als Ester und/oder Amid vorliegt,
V eine gegebenenfalls Imino-, Phenylen-, PhenylenoxyPhenylenimino-, Amid-, Hydrazid-, Ureido-, Thioureido-, Carbonyl-, Estergruppe(n), Sauerstoff-, Schwefel- und/oder Stickstoff-Atom(e) enthaltende gegebenenfalls durch Hydroxy-, Mercapto-, Imino-, Epoxy-, Oxo-, Thioxo- und/oder Aminogruppen (n) substituierte geradkettige, verzweigte, gesättigte oder ungesättigte C₁-C₂₀-Alkylengruppe bedeutet, wobei die Reproduktionseinheiten S nur für eine Generation identisch sein müssen und die Anzahl der Generationen 2 bis 10 beträgt, und wobei die Polymeren in den Komplexresten K mindestens fünf Ionen eines Elements der Ordnungszahlen 21 bis 29, 39, 42, 44 oder 57 bis 83 komplexiert sowie gegebenenfalls Kationen anorganischer und/oder organischer Basen, Aminosäuren oder Aminosäureamide enthalten,
zur Verwendung als Kontrastmittel für die NMR- oder Röntgen-Diagnostik.

Die gegebenenfalls für Z¹ und Z² stehenden Komplex(bildner)-Reste müssen nicht identisch sein.

Als beispielhaft genannte für Z¹ stehende Alkyl-, Acyl- und Alkylsulfonylreste seien aufgeführt: -CH₂COOH; -(CH₂)₂COOH; -CH(COOH)CH₂COOH; -CH₂-CH(COOH)CH₂OH; -CH₂SO₃H; -(CH₂)₂SO₃H; -COCH₃; -COCH₂OH; -COCHOHCH₂OH; -COCH₂O-CH₂COOH; -CO(CHOH)₄CH₂OH; -COCH₂COOH; -CO-(CH₂)₂COOH; -CO(CH₂)₃COOH; -CO(CH₂)₄COOH; -COCHOHCOOH; -CO(CHOH)₂COOH; -COCH₂CHOHCH₂COOH; -SO₂CH₂COOH; -SO₂(CH₂)₂COOH; -SO₂CH₃.

Als Kaskadenkern A sind geeignet:
Stickstoffatom
NR²R³R⁴, 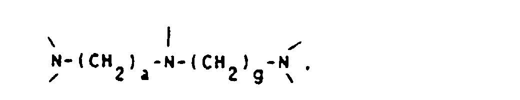 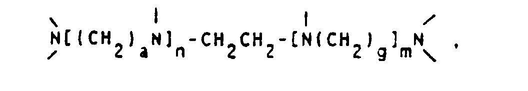 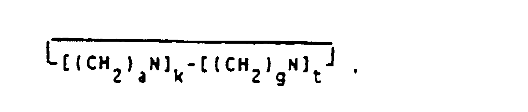 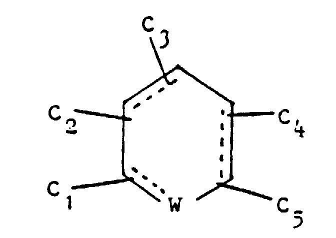 oder 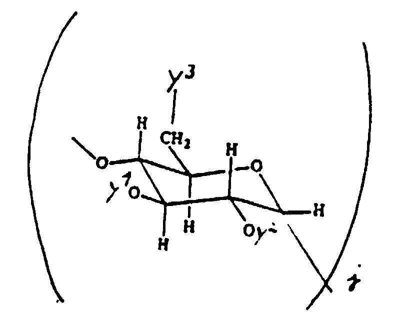 worin
- R², R³ und R⁴: jeweils unabhängig voneinander für eine kovalente Bindung oder (CH₂)ₖ-(C₆H₄)ᵣ-(CH₂)ₗ-N〈,
- g: für die Ziffern 2, 3, 4 oder 5,
- t: für die Ziffern 1, 2, 3, 4, 5, 6, 7 oder 8,
- W: für CH, CH₂, NH oder ein Stickstoffatom,
- C₁: für (CH₂)ₖ-N〈,
- C₂, C₃, C₄ und C₅: jeweils unabhängig für ein Wasserstoffatom oder (CH₂)_{f}-N〈,
- j: für die Ziffern 6, 7 oder 8,
- Y¹ und Y²: jeweils unabhängig voneinander für ein Wasserstoffatom, CH₂-CH(OH)-CH₂N〈 oder (CH₂)ₐ-N〈 und
- Y³: für ein Stickstoffatom, O-CH₂-CH(OH)-CH₂N〈 oder O-(CH₂)_{g}-N〈
- ---: für eine Einfach- oder Doppelbindung
stehen, mit der Maßgabe, daß wenn Y³ für ein Stickstoffatom steht, Y¹ und Y² für Wasserstoff stehen.

Den einfachsten Fall eines Kaskadenkerns stellt das Stickstoffatom dar, dessen drei Bindungen (Basismultiplizität b = 3) in einer ersten "inneren Schicht" (Generation 1) von drei Reproduktionseinheiten S, die jeweils 1 bis 3 terminale NH₂-Gruppen (s = 1-3) tragen, besetzt sind (bzw. die drei Wasserstoffatome des zugrundeliegenden Kaskadenstarters Ammoniak sind durch drei Einheiten S substituiert worden). Enthält die Reproduktionseinheit S z.B, eine NH₂-Gruppe (s = 1), so beträgt die Reproduktions-Multiplizität dieser Generation 2 s = 2. Die in einer nächsten Reaktionsfolge eingeführte 2. Schicht (Generation 2) von Reproduktionseinheiten S (die in dem oben genannten Beispiel mit A = Stickstoffatom und s = 1 sechs Bindungen besetzt) muß nicht identisch sein mit den Reproduktionseinheiten S der 1. Generation. Nach maximal 10, vorzugsweise 2 bis 6 Generationen sind die terminalen Stickstoffatome der äußersten Schicht wie oben für Z¹ und Z² der letzten Generation angegeben substituiert.

Als weitere bevorzugte Kaskadenstarter A(H)_{b} seien u.a aufgeführt:
Tris(aminoethyl)amin (b = 6);
Tris(aminopropyl)amin (b = 6);
Diethylentriamin (b = 5);
Triethylentetramin (b = 6);
Tetraethylenpentamin (b = 7);
H₂N-CH₂-C₆H₄-CH₂-NH-CH₂-C₆H₄-CH₂-NH₂ (b = 5);
1,3,5-Tris(aminomethyl)benzol (b = 6);
2,4,6-Tris(aminomethyl)-pyridin (b = 6);
1,4,7-Triazacyclononan (b = 3);
1,4,7,10-Tetraazacyclododecan (b = 4);
1,4,7,10,13-Pentaazocyclopentadecan (b = 5);
1,4,8,11-Tetraazacyclotetradecan (b = 4);
1,4,7,10,13,16,19,22,25,28-Decaazacyclotriacontan (b = 10);
6,6',6",6"',6"",6""'-Hexaamino-6,6',6",6"',6"",6'""-Hexadeoxy-α-cyclodextrin (b = 12);
6,6',6'',6"',6"",6""'-Heptaamino-6,6',6",6"',6"",6""'-Heptadeoxy-β-cyclodextrin (b = 14);
6,6',6",6"',6"",6""'-Hexa-(1-Amino-2-hydroxypropyl)-α-cyclodextrinhexaether (b = 12);
2,2',2",2"',2"",2""',6,6',6",6"',6"",6""'-Dodeca-(1-Amino-2-hydroxypropyl)-α-cyclodextrin-dodeca-ether (b = 24).

Eine Reproduktionseinheit S kann eine solche entsprechend den Formeln
-(CH₂)₂-CONH-(CH₂)ₐ-
oder 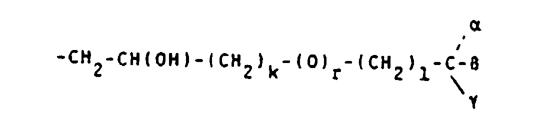 sein worin
- α und β: jeweils für ein Wasserstoffatom oder (CH₂)ₒ,
- γ: für (CH₂)_{f},
- f: für die Ziffern 1, 2, 3, 4 oder 5,
- o: für die Ziffern 0, 1, 2, 3, 4 oder 5, wobei l und o nicht gleichzeitig für die Ziffer 0 stehen sollen, und
- r: für die Ziffern 0 oder 1
stehen.

Bevorzugte Reproduktionseinheiten S sind
-(CH₂)₂-CONH-(CH₂)₂-;
-CH₂-CH(OH)-CH₂-;
-CH₂-CH(OH)-CH₂-O-(CH₂)₂-;

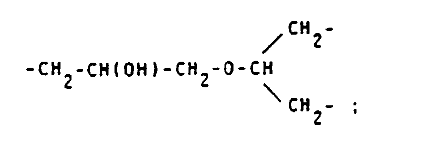

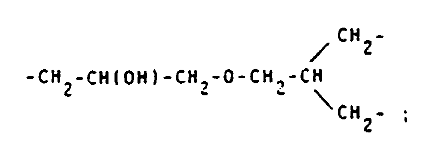

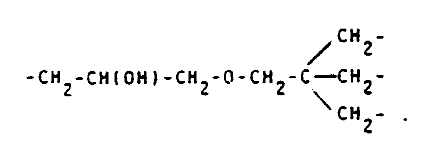

Als Beispiele für die Komplexbildner-Reste K seien diejenigen der 1,4,7,10-Tetraazacyclododecantetraessigsäure, 1,4,7-Triazacyclononan-triessigsäure, 1,4,8,11-Tetraazatetradecantetraessigsäure, 1,5,9-Triazacyclododecantriessigsäure, 1,4,7,10-Tetraazacyclododecantriessigsäure genannt, die über (jeweils in K enthaltend) eine Carbonylgruppe, wenn gleichzeitig R¹ für ein Wasserstoffatom steht) oder über ein (in V enthaltenes, s. die Definition für U und R¹) Kohlenstoffatom, (wenn gleichzeitig U für CH₂X steht) an jeweils eine terminale -NH₂-Gruppe der letzten Generation des Kaskaden-Polymeren gebunden sind. Gewünschtenfalls kann ein Teil der Carbonsäuren als Ester und/oder Amid vorliegen.

Ist das erfindungsgemäße Mittel zur Anwendung in der NMR-Diagnostik bestimmt, so muß das Zentralion des Komplexsalzes paramagnetisch sein. Dies sind insbesondere die zwei- und dreiwertigen Ionen der Elemente der Ordnungszahlen 21 - 29, 42, 44 und 58 - 70. Geeignete Ionen sind beispielsweise das Chrom(III)-, Mangan(II)-, Eisen(II)-, Cobalt(II)-, Nickel(II)-, Kupfer(II)-, Praseodym(III)-, Neodym(III)-, Samarium(III)- und Ytterbium(III)-ion. Wegen ihres sehr starken magnetischen Moments sind besonders bevorzugt das Gadolinium(III)-, Terbium(III)-, Dysprosium(III)-, Holmium(III)-, Erbium(III)- und Eisen(III)-ion.

Ist das erfindungsgemäße Mittel zur Anwendung in der Röntgen-Diagnostik bestimmt, so muß sich das Zentralion von einem Element höherer Ordnungszahl ableiten, um eine ausreichende Absorption der Röntgenstrahlen zu erzielen. Es wurde gefunden, daß zu diesem Zweck diagnostische Mittel, die ein physiologisch verträgliches Komplexsalz mit Zentralionen von Elementen der Ordnungszahlen zwischen 21 - 29, 39, 42, 44, 57 - 83 enthalten, geeignet sind; dies sind beispielsweise das Lanthan(III)-ion und die oben genannten Ionen der Lanthanidenreihe.

Die erfindungsgemäßen Kaskaden-Polymer-Komplexe enthalten mindestens fünf Ionen eines Elements der oben genannten Ordnungszahl.

Die für V stehende Alkylengruppe sowie die für R und R' (s. weiter unten) stehende Alkylgruppe kann geradkettig, verzweigt, cyclisch, aliphatisch, aromatisch oder arylaliphatisch sein und bis zu 20 Kohlenstoffatome aufweisen. Bevorzugt sind geradkettige Mono- bis Decamethylengruppen sowie C₁-C₄-Alkylenphenylgruppen. Zur Verdeutlichung seien folgende Alkylengruppen beispielhaft genannt:
-CH₂-CH(OH)-CH₂-O-CH₂CH₂-;
-CH₂-CH(OH)-CH₂-; 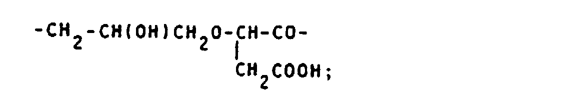 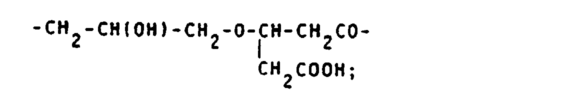 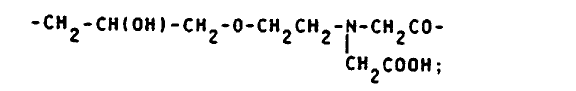 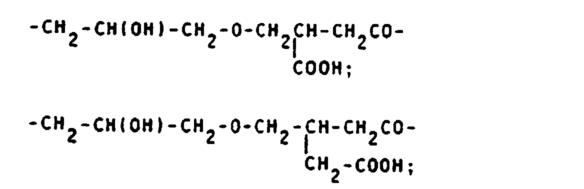 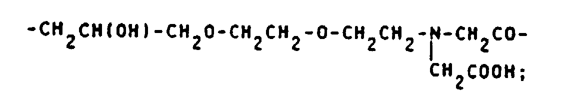 -CH₂-CH(OH)-CH₂-OCH₂-C₆H₄-NH-CO-CH₂-;
-CH₂-CH(OH)-CH₂-OCH₂-C₆H₄-NH-CO-CH₂-S-(CH₂)₄-S-; 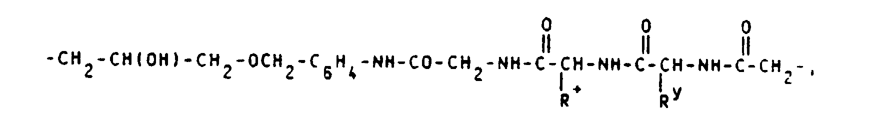 worin R⁺ und R^{y} für natürliche Aminosäurereste stehen;
-CH₂-OH(OH)-CH₂-O-(CH₂)₂-NHCS-;
-CH₂-CH(OH)-CH₂-NHCS-;
-CH₂-CH(OH)-CH₂-O-(CH₂)₂-O-(CH₂)₂NHCS-;
-CH₂-CH(OH)-CH₂-O-(CH₂)₂-NH-CO-CH₂-;
-CH₂-CH(OH)-CH₂-O-C₆H₄-NHCS-
-CH₂-CH(OH)-CH₂-O-C₆H₄-NHCO-;
-CH₂-CH(OH)-CH₂-O-CH₂-C₆H₄-NHCS-;
-CH₂-O-C₆H₄-CH₂-; -CH₂-CH(OH)-CH₂-O-C₆H₄-CH₂-; -C(= NH)-O-C₆H₄-CH₂-;
-(CH₂)₄-NH-CO-CH₂-O-C₆H₄-CH₂-; -(CH₂)₄-NH-CH₂-CH(OH)-CH₂-O-C₆H₄-CH₂-;
-(CH₂)₃-O-C₆H₄-CH₂-; -CH₂-CO-NH-(CH₂)₃-O-CH₂-; -CH₂-CO-NH-NH-;
-CH₂-CONH-(CH₂)₂-; -CH₂-CO-NH-(CH₂)₁₀-; -CH₂-CONH-(CH₂)₂-S-;
-(CH₂)₄-NH-CO-(CH₂)₈-; -CH₂-CO-NH-(CH₂)₃-NH-; -(CH₂)₃-NH-Gruppe steht.

Die restlichen aciden Wasserstoffatome, das heißt diejenigen, die nicht durch das Zentralion substituiert worden sind, können gegebenenfalls ganz oder teilweise durch Kationen anorganischer und/oder organischer Basen oder Aminosäuren ersetzt sein. Die entsprechenden Säuregruppen können auch ganz oder teilweise in Ester oder Amide überführt sein.

Geeignete anorganische Kationen sind beispielsweise das Lithiumion, das Kaliumion, das Calciumion, das Magnesiumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins sowie die Amide ansonsten saurer oder neutraler Aminosäuren.

Geeignete Ester sind vorzugsweise diejenigen mit einem C₁-C₆-Alkylrest; genannt seien beispielsweise der Methyl-, Ethyl- und tertiär-Butylrest.

Sollen die Carbonsäuregruppen zumindest teilweise als Amide vorliegen, so sind tertiäre Amide bevorzugt. Als Reste kommen gesättigte, ungesättigte, gerad- oder verzweigtkettige oder cyclische Kohlenwasserstoffe mit bis zu 5 C-Atomen infrage, die gegebenenfalls durch 1 bis 3 Hydroxy- oder C₁-C₄-Alkoxy-Gruppen substituiert sind. Beispielsweise genannt seien die Methyl-, Ethyl-, 2-Hydroxyethyl-, 2-Hydroxy-1-(hydroxymethyl)-ethyl-, 1-(Hydroxymethyl)-ethyl-, Propyl-, Isopropenyl-, 2-Hydroxypropyl-, 3-Hydroxypropyl-, 2,3-Dihydroxypropyl-, Butyl-, Isobutyl-, Isobutenyl-, 2-Hydroxybutyl-, 3-Hydroxybutyl-, 4-Hydroxybutyl-, 2-, 3- und 4-Hydroxy-2-methylbutyl-, 2- und 3-Hydroxyisobutyl-, 2,3,4-Trihydroxybutyl-, 1,2,4-Trihydroxybutyl-, Pentyl-, Cyclopentyl- und 2-Methoxyethyl-gruppe. Der Amidrest kann auch ein unter Einschluß des Amid-Stickstoffs gebildeter heterocyclischer 5- oder 6-Ring sein. Beispielhaft genannt seien: der Pyrrolidinyl-, Piperidyl-, Pyrazolidinyl-, Pyrrolinyl-, Pyrazolinyl-, Piperizinyl-, Morpholinyl-, Imidazolidinyl-, Oxazolidinyl-, Thiazolidinyl-Ring.

Die erfindungsgemäßen Verbindungen weisen die eingangs geschilderten gewünschten Eigenschaften auf. Sie enthalten die für ihre Verwendung benötigte große Anzahl von Metallionen im Komplex stabil gebunden. Sie verteilen sich nur im vasalen Raum und können daher diesen mit Hilfe der Kernspintomographie markieren.

Die Verträglichkeit der erfindungsgemäßen Verbindungen ist gegenüber Magnevist um mindestens den Faktor 3 besser (LD₅₀ i.v. Maus von Beispiel 8: 30 mmol/kg: Magnevist:≦ 10).

Der für Nebenwirkungen wie Schmerzen, Schädigungen der Blutgefäße und Herz-Kreislauf-Störungen verantwortliche Wert der Osmolalität ist im Vergleich zu Magnevist deutlich verringert (Beispiel 8: 0,46 [osmol/kg] gegenüber Magnevist 1.96 [osmol/kg], 0,5 mol/l bei 37 °C).

Überraschend groß ist der Wert für die ein Maß der Bildgebung beim MRI darstellende Große der Relaxivity; die Signalverstärkung ließ sich z.B. im Falle der Verbindung des Beispiels 8 gegenüber Magnevist um das 4fache steigern.

Im Vergleich zu den makromolekularen Kontrastmitteln auf der Basis von Kohlenhydraten, z.B. Dextran (Europäische Patentanmeldung, Publikations-Nr. 0 326 226), die - wie erwähnt - in der Regel nur 4,6 % des signalverstärkenden paramagnetischen Kations tragen, weisen die erfindungsgemäßen Polymer-Komplexe einen Gehalt von über 15 % des paramagnetischen Kations auf. Somit bewirken die erfindungsgemäßen Makromoleküle pro Molekül eine sehr viel höhere Signalverstarkung, was gleichzeitig dazu führt, daß die zur Kernspintomographie notwendige Dosis gegenüber der makromolekularer Kontrastmittel auf Kohlenhydratbasis erheblich kleiner ist.

Mit den erfindungsgemäßen Polymer-Komplexen ist es gelungen, Makromoleküle so zu konstruieren und herzustellen, daß diese ein einheitlich definiertes Molekularyewicht haben. Solche, in ihrer Molekulgröße exakt definierbare makromolekulare Kontrastmittel waren bislang nicht zugänglich. Es ist somit überraschenderweise erstmals möglich, die Größe der Makromoleküle so zu steuern, daß diese groß genug sind, um den vasalen Raum nur langsam verlassen zu können, aber gleichzeitig klein genug, die Kapillaren der Niere, welche 300 - 800 Å groß sind, noch passieren zu können. Damit ist es erstmals möglich, makromolekulare Kontrastmittel körpergerecht herzustellen.

Die erfindungsgemaßen Komplexe dienen als Kontrastmittel zur Darstellung der Gefäße mittels der Kernspintomographie. Es ist damit möglich, ischämisches Gewebe von normalem Gewebe zu unterscheiden. Aber auch andere Schädigungen der Blut-Gewebe-Schranke sind mit diesen Verbindungen zu erkennen. Bei Entzündungen und Tumoren im Gehirn ist die Blut-Hirn-Schranke so geschädigt, daß das Kontrastmittel das kranke Gewebe infiltrieren kann und somit das kranke Gewebe in der Kernspintomographie erkennbar wird. Aufgrund der Undurchlässigkeit der intakten Blut-Hirn-Schranke auch für kleine, aber hydrophile Moleküle konnte man Entzündungen und Tumore auch schon mit der niedermolekularen Verbindung Magnevist erkennen. Wendet man in diesen Fallen aber die erfindungsgemäßen Komplexe an, kann man aus zwei Gründen die Dosis um das 16fache reduzieren: 1. sie haben eine 4fach höhere Signalverstärkung und 2. verteilen sie sich in einem 4fach kleineren Raum, nämlich nur im Vasalraum, d.h. um gleiche Konzentrationen im Blut zu erreichen, genügt ein Viertel der Dosis.

Ein weiterer Vorteil der vorliegenden Erfindung liegt darin, daß nunmehr Komplexe mit hydrophilen oder lipophilen, makrocyclischen oder offenkettigen, niedermolekularen oder hochmolekularen Liganden zugänglich geworden sind. Dadurch ist die Möglichkeit gegeben. Verträglichkeit und Pharmakokinetik dieser Polymer-Komplexe durch chemische Substitution zu steuern.

Die Herstellung der erfindungsgemäßen Kaskaden-Polymere erfolgt dadurch, daß man Verbindungen der allgemeinen Formel I' 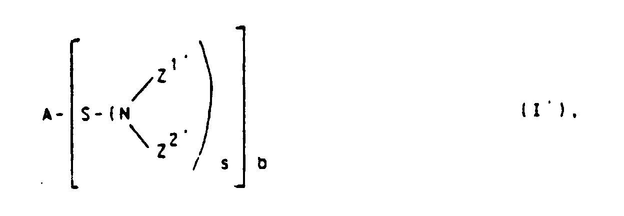 worin
- A: für einen Stickstoff-haltigen Kaskadenkern der Basismultiplizität b,
- S: für eine Reprodukfionseinheit,
- N: für ein Stickstoffatom,
- Z^{1'} und Z^{2'}: für die erste bis vorletzte Generation jeweils für 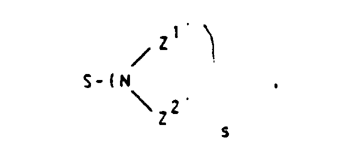 für die letzte Generation dagegen jeweils für ein Wasserstoffatom,
- b: für die Ziffern 1 bis 50 und
- s: für die Ziffern 1 bis 3
stehen, wobei die Reproduktionseinheiten S nur für eine Generation identisch sein müssen mit einem Komplex oder Komplexbildner K^{'} der allgemeinen Formel 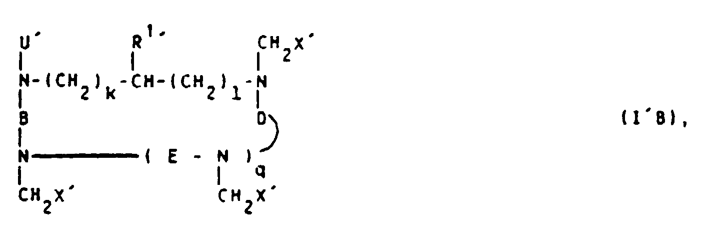 wobei
- k: für die Ziffern 1, 2, 3, 4 oder 5,
- l: für die Ziffern 0, 1, 2, 3, 4 oder 5,
- q: für die Ziffern 0, 1 oder 2,
- U': für -CH₂C*O-, CH₂X' oder V", wobei V" für eine gegebenenfalls Imino-, Phenylen-. Phenylenoxy-, Phenylenimino-, Amid-, Hydrazid-, Ureido-. Thioureido-, Carbonyl-, Estergruppe(n), Sauerstoff-, Schwefel- und oder Stickstoff-Atom(e) enthaltende gegebenenfalls durch Hydroxy-, Mercapto-, Imino-, Epoxy-, Oxo-, Thioxo- und/oder Aminogruppe(n) substituierte geradkettige, verzweigte, gesättigte oder ungesättigte C₁-C₂₀-Alkylengruppe
steht, die am Ende eine funktionelle Gruppe trägt.
- X': unabhängig voneinander jeweils für die Reste -COOH, COOY oder V"' steht,
worin
- Y: eine Säureschutzgruppe oder ein Metallonenäquivalent eines Elements der Ordnungszahlen 21-29, 39, 42, 44 oder 57-83 und
- V"': einen in V' umzuwandelnden Substituenten bedeutet,
- C*O: für eine aktivierte Carbonylgruppe
- B, D und E,: die gleich oder verschieden sind, jeweils für die Gruppe (CH₂)ₐ mit a in der Bedeutung der Ziffern 2, 3, 4 oder 5,
- R¹ für V": oder ein Wasserstoffatom
stehen,
mit der Maßgabe, daß R^{1'} nur dann für V" steht, wenn U' gleichzeitig CH₂X' bedeutet, und daß U' nur dann für -CH₂C*O- oder V" steht, wenn gleichzeitig R¹ ein Wasserstoffatom bedeutet,
umsetzt, gegebenenfalls vorhandene Schutzgruppen gewünschtenfalls abspaltet, die so erhaltenen Kaskaden-Polymere gewünschtenfalls - sofern K' für einen Komplexbildner steht - in an sich bekannter Weise mit mindestens einem Metalloxid oder Metallsalz eines Elements der Ordnungszahlen 21 - 29, 39, 42, 44 oder 57 - 83 umsetzt und in beliebiger Reihenfolge gegebenenfalls anschließend in den so erhaltenen Polymer-Komplexen noch vorhandene acide Wasserstoffatome ganz oder teilweise durch Kationen anorganischer und/oder organischer Basen, Aminosäuren oder Aminosäureamide substituiert bzw. die entsprechenden Säuregruppen ganz oder teilweise in Ester oder Amide überführt.

Als Beispiel für eine aktivierte Carbonylgruppe in den Komplexen bzw. Komplexbildnern K' seien Anhydrid, p-Nitrophenylester und Säurechlorid genannt.

Die zur Einführung der Komplexbildner-Einheiten vorgenommene Alkylierung bzw. Acylierung wird mit Substraten durchgeführt die den gewünschten Substituenten K (eventuell gebunden an eine Fluchtgruppe) enthalten, oder aus denen der gewünschte Substituent, gegebenenfalls nach Modifikation durch Folgereaktion(en), durch die Reaktion generiert wird. Als Beispiele für die erstgenannten seien Halogenide, Mesylate, Tosylate und Anhydride genannt. Zur zweiten Gruppe gehören zum Beispiel Oxirane, Thiirane, Azirane, α,β-ungesättigte Carbonylverbindungen oder deren Vinvloge, Aldehyde, Ketone, Isothiocyanate und Isocyanate.

Als Beispiele für Folgereaktionen seien Esterspaltungen, Hydrierungen, Veresterungen, Oxidationen, Veretherungen und Alkylierungen genannt, die nach dem Fachmann bekannten Literaturverfahren durchgeführt werden.

Als ausgewählte Beispiele für die in K' enthaltenen Reste V" seien genannt:
-CH₂-CHOH-CH₂-NCS;
-CH₂-CHOH-CH₂-O-(CH₂)₂-NCS;
-CH₂-CHOH-CH₂-O-(CH₂)₂-NH-CO-CH₂-Br;
-CH₂-CHOH-CH₂-O-(CH₂)₂-O-(CH₂)₂-NCS;
-NCS;
-CH₂-CHOH-CH₂-O-CH₂-CHO;
-CH₂-CHOH-CHO; 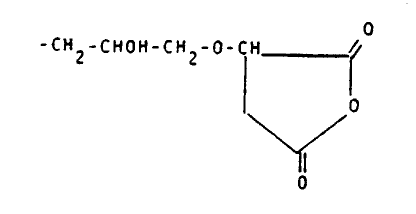 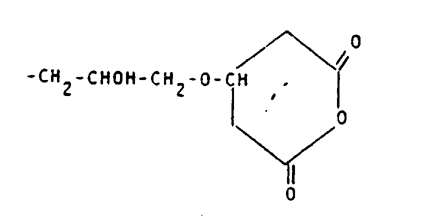 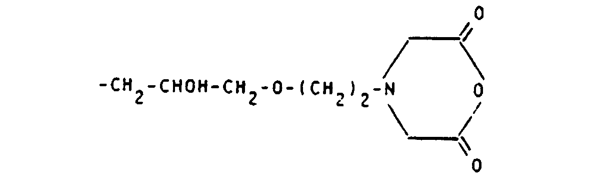 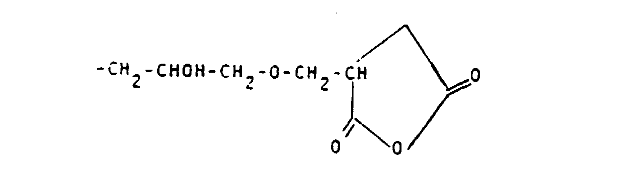 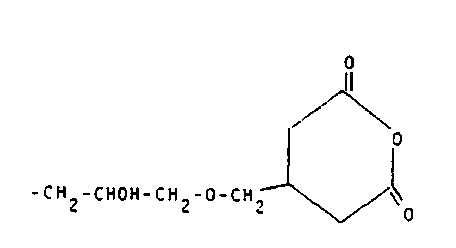 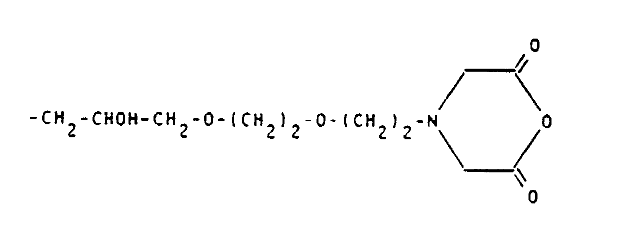 Beispielhaft genannt seien die Umsetzung des Monoanhydrids N³⁻(2,6-Dioxomorpholinoethyl)-N⁶(ethoxycarbonylmethyl)-3,6-diazaoctandisäure mit den jeweils gewünschten, terminale Aminogruppen enthaltenden Kaskaden-Polymeren in Wasser oder in Mischungen von Wasser mit z.B. Dioxan, THF, DMF, DMSO oder Acetonitril bei basischem pH, vorzugsweise 8 - 10, d.h. unter Zusatz von Basen wie z.B. Natrium-. Kaliumhydroxid oder Triethylamin, bei Temperaturen von 0 - 50 ° C, vorzugsweise bei Raumtemperatur. Zur vollständigen Umsetzung arbeitet man vorzugsweise mit einem z.B. 2 - 3fachen Überschuß Monoanhydrid.

Als weitere Möglichkeit sei die Umsetzung von endständige Aldehydgruppen aufweisenden Substituenten K' mit den jeweils gewünschten, terminale Aminogruppen enthaltenden Kaskaden-Polymeren mit anschließender Reduktion der dabei entstehenden Schiff-Basen analog literaturbekannten Methoden [Synthesis 1975, 135) genannt. Die hierbei generierten sekundären Amine können durch nachfolgende Acylierung oder Alkylierung mit gegebenenfalls 1 bis 3 Carboxyl-, 1 bis 3 Sulfonsäure-, 1 bis 5 Hydroxyreste und/oder 1 bis 3 Sauerstoffatome enthaltenden α,β-ungesättigten Estern, Alkylhalogeniden, Anhydriden, Säurehalogeniden oder Komplexen bzw. Komplexbildnern K' in tertiäre Amine, Amide oder Thioamide überführt werden. Als beispielhaft genannte, die sekundären Amino-Wasserstoffatome substituierenden Reaktionspartner seien aufgeführt:
Br-CH₂COOH; Cl-CH₂-CH₂-COOH; H₂C=CH-COOCH₃; HOOC-CH = CH-COOCH₃; CH₂ = C(OOOEt)-CH₂OH; Br-CH₂-So₃H; Cl-CH₂-CH₂-SO₃H; CH₃-CO-Cl; Cl-CO-CH₂OH; Cl-CO-CHOH-CH₂OH; ClSO₂CH₃; ClSO₂CH₂COOC₂H₅; Br-CO-(CHOH)₄-CH₂OH; Cl-CO-CH₂-COOC₂H₅; 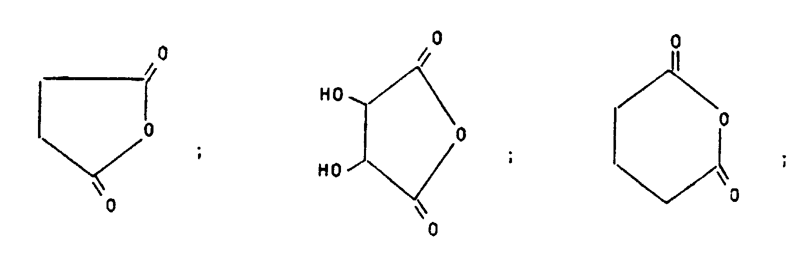 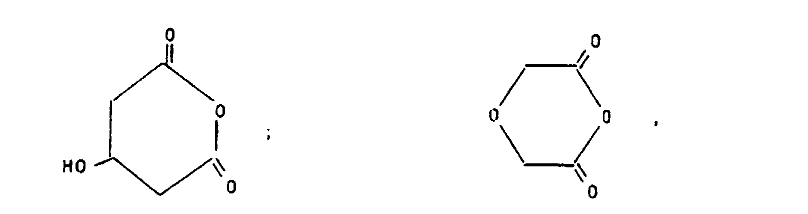 Br-CO-(CH₂)₄-COOC(CH₃)₃; Cl-CO-CHOH-COOH₃.

Die dabei als Edukte benötigten Aldehyde können aus den entsprechenden vicinalen Diolen durch Oxidation mit beispielsweise Natriummetaperjodat in wäßriger oder alkoholischer Losung analog literaturbekannten Methoden (z.B. Makromol Chem. 182, 1641 [1981]) hergestellt werden.

Durch geeignete Reaktionsführung, z. B. Einstellung des pH-Werts oder Zusatz von Aminen, können miteingeführte Estergruppen gewünschtenfalls verseift bzw. aminolysiert werden.

Die Reinigung der so erhaltenen Kaskaden-Polymere erfolgt vorzugsweise durch Ultrafiltration mit Membranen geeigneter Porengröße (z. B. Amicon^{(R)}) oder Gelfiltration an z.B. geeigneten Sephadex^{(R)}-Gelen.

In analoger Weise werden z.B. isothiocyanat-, epoxid- oder α-halogenacetylderivatisierte Komplexbildner bzw. Komplexe unter pH-Kontrolle in wäßrigem Milieu mit den gewünschten Kaskaden-Polymer-Aminen zur Reaktion gebracht.

Die als Edukte benötigten Verbindungen I' sind bekannt (z.B. Europäische Patentanmeldungen, Publikationsnummern 0 154 788 und 0 331 616, Deutsche Patentanmeldung P 38 25 040.3) oder können aus den entsprechenden Polyaminen (wobei vorhandene funktionelle Gruppen gegebenenfalls geschützt sind) durch Alkylierung mit einem Ester der allgemeinen Formel II

HalCH₂COOY' (II),

worin Hal für Chlor, Brom oder Jod und Y' für ein Wasserstoffatom, ein Alkalimetall oder eine Säureschutzgruppe Y steht, hergestellt werden.

Die Umsetzung erfolgt in polaren aprotischen Lösungsmitteln wie zum Beispiel Dimethylformamid, Dimethylsulfoxid, Acetonitril, wäßriges Tetrahydrofuran oder Hexamethylphosphorsäuretriamid in Gegenwart eines Saurefängers, wie zum Beispiel tertiares Amin (zum Beispiel Triäthylamin, Trimethylamin, N,N-Dimethylaminopyridin, 1,5-Diazabicyclo[4.3.0]-nonen-5(DBN), 1,5-Diazabicyclo[5.4.0]-undecen-5-(DBU), Alkali-, Erdalkalicarbonat, -hydrogencarbonat oder Hydroxid (zum Beispiel Natrium-, Magnesium-, Calcium-, Barium-, Kalium-, -carbonat, -hydroxid und -hydrogencarbonat) bei Temperaturen zwischen -10 °C und 120 °C, vorzugsweise zwischen 0 °C und 50 °C.

Als Säureschutzgruppen Y kommen niedere Alkyl-, Aryl- und Aralkylgruppen, beispielsweise die Methyl-, Ethyl-, Propyl-, Butyl-, Phenyl-, Benzyl-, Diphenylmethyl-, Triphenylmethyl-, bis-(p-Nitrophenyl)-methylgruppe, sowie Trialkylsilylgruppen infrage.

Die gegebenenfalls gewünschte Abspaltung der Schutzgruppen Y erfolgt nach den dem Fachmann bekannten Verfahren, beispielsweise durch Hydrolyse, Hydrogenolyse, alkalische Verseifung der Ester mit Alkali in wäßrig-alkoholischer Losung bei Temperaturen von 0 °C bis 50 °C oder im Fall von tert.-Butylestern mit Hilfe von Trifluoressigsäure.

Die Herstellung der Derivate mit aktivierter Carbonylgruppe C*O, I'A bzw. I'B und I'C mit U' in der Bedeutung von CH₂C*O (z.B. gemischtes Anhydrid, N-Hydroxysuccinimidester, Acylimidazole, Trimethylsilylester) erfolgt nach literaturbekannten Methoden [Houben-Weyl, Methoden der organischen Chemie, Georg Thieme Verlag, Stuttgart, Band E 5 (1985), 633; Org. React. 12, 157 (1962] oder wird im experimentellen Teil beschrieben.

Die Herstellung der als Edukte für I'B benötigten cyclischen Polyamine erfolgt durch Cyclisierung zweier Reaktanten, von denen - im Falle der Synthese von I'B mit R^{1'} = V" - der eine V'"-subsbtuiert ist.

Die Cyclisierung wird nach literaturbekannten Methoden [zum Beispiel Org. Synth. 58, 86 (1978). Makrocyclic Polyether Syntheses, Springer Verlag Berlin, Heidelberg, New York (1982), Coord. Chem. Rev. 3, 3 (1968), Ann. Chem. 1976. 916, J. Org. Chem. 49, 110 (1984)] durchgeführt; einer der beiden Reaktanten tragt am Kettenende zwei Fluchtgruppen, der andere zwei Stickstoffatome, die nukleophil diese Fluchtgruppen verdrängen. Als Beispiel seien genannt die Umsetzung von endständigen, gewünschtenfalls den Substituenten V"' und gegebenenfalls ein bis fünf Stickstoffatom(e) enthaltenden Dichlor-, Dibrom-, Dimesyloxy-, Ditosyloxy- oder Dialkoxycarbonylalkylenverbindungen mit endstandigen, gegebenenfalls ein bis fünf zusätzliche Stickstoffatom(e) in der Alkylenkette enthaltenden Polyazaalkylenverbindungen. Der Substituent V"' kann stattdessen auch in dem zweiten Reaktanten, d.h. dem mit den endständigen nukleophilen Stickstoffatomen, enthalten sein. Die Stickstoffatome sind gegebenenfalls geschützt, zum Beispiel als Tosylate oder Trifluoracetate, und werden vor der nachfolgenden Alkylierungsreaktion nach literaturbekannten Verfahren freigesetzt (die Tosylate z.B. mit Mineralsauren, Alkalimetallen in flüssigem Ammoniak, Bromwasserstoffsaure und Phenol, RedAl^{(R)}, Lithiumaluminiumhydrid, Natrium-Amalgam, vgl. z.B. Liebigs Ann. Chem. 1977. 1344, Tetrahedron Letters 1976, 3477; die Trifluoracetate z.B. mit Mineralsauren oder Ammoniak in Methanol, vgl. z.B. Tetrahedron Letters 1967, 289).

Zur Herstellung von an den Stickstoffatomen unterschiedlich (Wasserstoff oder die Gruppe CH₂COOY) substituierten Makrocyclen können diese Atome in den Edukten mit unterschiedlichen Schutzgruppen, z.B. mit Tosylat- und Benzylgruppen, versehen werden. Letztere werden dann ebenfalls nach literaturbekannten Methoden (bevorzugt durch Hydrierung, z.B. EP-Patentanmeldung 232 751) entfernt.

Werden Diester in die Cyclisierungsreaktion eingesetzt, so müssen die so erhaltenen Diketoverbindungen nach dem Fachmann bekannten Verfahren, zum Beispiel mit Diboran, reduziert werden.

Es können auch entsprechend substituierte endständige Bisaldehyde mit den jeweils gewünschten endständigen Bisaminen cyclisiert werden; die Reduktion der so erhaltenen Schiffschen Basen erfolgt nach literaturbekannten Methoden, z.B. durch katalytische Hydrierung [Helv. Chim. Acta 61, 1376 (1978)].

Die Herstellung der als Ausgangssubstanzen für die Cyclisierung benötigten Amine erfolgt analog literaturbekannten Methoden.

Ausgehend von einer N-geschutzten Aminosäure erhält man durch Umsetzung mit einem partiell geschützten Diamin (zum Beispiel nach der Carbodiimidmethode), Abspaltung der Schutzgruppen und Diboranreduktion ein Triamin.

Die Umsetzung eines aus Aminosäuren erhältlichen Diamins (Eur. J. Med. Chem.-Chim. Ther. 21, 333 (1986)] mit der doppelten molaren Menge einer N-geschützten ω-Aminosäure liefert ein Tetramin nach geeigneter Aufarbeitung.

Die gewünschten Diamine kenne auch durch Gabriel-Reaktion aus z.B. den entsprechenden Tosylaten oder Halogeniden hergestellt werden [vgl. z.B. Inorg. Chem. 25, 4781 (1986)].

In beiden Fällen ist die Anzahl der Kohlenstoffatome zwischen den N-Atomen durch die Art der als Kopplungspartner eingesetzten Diamine bzw. Aminosäuren bestimmbar.

Als Substituenten V"', der in V oder in den am Ende eine für eine Bindung an ein Kaskaden-Polymeres geeignete funktionelle Gruppe aufweisenden Substituenten V" überführt werden kann, sind unter anderem Hydroxy- und Nitrobenzyl-, Hydroxy- und Carboxyalkyl- sowie Thioalkylreste mit bis zu 20 Kohlenstoffatomen geeignet. Sie werden nach dem Fachmann bekannten Literaturverfahren [Chem. Pharm. Bull. 33, 674 (1985), Compendium of Org. Synthesis Vol. 1-5, Wiley and Sons, Inc., Houben-Weyl, Methoden der organischen Chemie, Band VIII, Georg Thieme Verlag, Stuttgart, J. Biochem. 92, 1413, (1982)] in die gewünschten Substituenten (zum Beispiel mit der Amino-, Hydrazino-, Hydrazinocarbonyl-, Epoxid-, Anhydrid-, Methacryloylhydrazinocarbonyl-, Maleimidamidocarbonyl-, Halogeno-, Halogenocarbonyl-, Mercapto-, Isothiocyanatgruppe als funktioneller Gruppe) umgewandelt, wobei im Falle des Nitrobenzylrestes zunächst eine katalytische Hydrierung (zum Beispiel nach P.N. Rylander, Catalytic Hydrogenation over Platinum Metals, Academic Press 1967) zum Aminobenzylderivat vorgenommen werden muß.

Beispiele für die Umwandlung von an aromatische oder aliphatische Reste gebundenen Hydroxy- oder Aminogruppen sind die in geeigneten Losungsmitteln wie Tetrahydrofuran, Dimethoxyethan oder Dimethylsulfoxid, zweiphasigen wäßrigen Systemen, wie z.B. Wasser/Dichlormethan, in Gegenwart eines Säurefangers wie zum Beispiel Natriumhydroxid, Natriumhydrid oder Alkali oder Erdalkalicarbonaten wie zum Beispiel Natrium-, Magnesium-, Kalium-, Calciumcarbonat oder Poly-(4-vinylpyridin) Reillex^{(R)} bei Temperaturen zwischen 0 °C und dem Siedepunkt des jeweiligen Losungsmittels, vorzugsweise jedoch zwischen 20 °C und 60 °C, durchgeführten Umsetzungen mit einem Substrat der allgemeinen Formel III

Nf-L-Fu (III),

worin Nf für ein Nucleofug wie z.B. Cl, Br, J. CH₃C₆H₄SO₃ oder CF₃SO₃, L für einen aliphatischen, aromatischen, arylaliphatischen, verzweigten, geradkettigen oder cyclischen Kohlenwasserstoffrest mit bis zu 20 Kohlenstoffatomen und Fu für die gewünschte endständige funktionelle Gruppe, gegebenenfalls in geschützter Form, stehen (DE-OS 34 17 413).

Als Beispiele für Verbindungen der allgemeinen Formel III seien genannt Br(CH₂)₂NH₂, Br(CH₂)₃OH, BrCH₂COOCH₃, BrCH₂CO₂^{t}Bu, ClCH₂CONHNH₂, Br(CH₂)₄CO₂C₂H₅, BrCH₂COBr, BrCH₂CONH₂, ClCH₂COOC₂H₅, BrCH₂CONHNH₂, 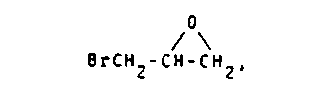 CF₃SO₃(CH₂)₃Br, BrCH₂C≡H, BrCH₂CH=CH₂.

Umwandlungen von Carboxy-Gruppen können zum Beispiel nach der Carbodiimid-Methode (Fieser, Reagents for Organic Syntheses 10, 142), über ein gemischtes Anhydrid [Org. Prep. Proc. Int. 7, 215 [1975/] oder über einen aktivierten Ester [Adv. Org. Chem. Part B, 472) durchgeführt werden.

Die Einführung des gegebenenfalls gewünschten Substituenten V" an ein Stickstoffatom der Komplexbildner I'B (d.h. U' = V") kann ebenfalls nach dem oben angegebenen Verfahren vorgenommen werden, d.h. es wird auch hier in der Regel eine V"'-enthaltende Makrocyclus-Vorstufe, die durch Umsatz eines nur eine freie NH-Gruppe aufweisenden Polyaza-Makrocyclus erhalten wird, durchlaufen. Beispielsweise genannt seien die Umsetzung von z.B. 1,4,7-Triscarboxymethyl-1,4,7,10-tetraazacyclododecan mit einem eine geschutzte Aminogruppe aufweisenden primären Epoxid, anschließende Freisetzung der Aminofunktion des so erhaltenen V"'-substituierten Makrocyclus und nachfolgende Umwandlung in einen V"-substituierten Makrocyclus (z.B. Überführung der Aminogruppe in eine an das Kaskaden-Polymer-Amin kopplungsfahige funktionelle Gruppe wie z.B. die Isothiocyanat- oder 2-Halogenacetamidgruppe).

Die Herstellung der für die Kopplung an die Komplexbildner K (bzw. auch die entsprechenden metallhaltigen Komplexe) benötigten terminalen Aminogruppen tragenden Kaskaden-Polymere erfolgt nach dem Fachmann bekannten Methoden durch kaskadenartige, generationsweise Einführung von Stickstoffatomen in ein stickstoffhaltiges Basismolekul. Dabei entsteht eine Generation aus mindestens zwei Reaktionsschritten. So werden aus jedem Aminowasserstoffatom des Kaskadenstarters durch z.B. eine Michael-Addition oder Anlagerung eines eine geeignete funktionelle Gruppe enthaltenden primären Epoxids und nachfolgende Umwandlung der so eingeführten funktionellen Gruppen bis zu drei Aminogruppen generiert.

Als Beispiel sei die Substitution der sechs Aminowasserstoffatome des Kaskadenstarters Tris-(aminoethyl)amins durch sechs -CH₂CH₂-CONH-CH₂CH₂NH₂-Einheiten genannt, die durch Michael-Addition mit Acrylsäureester und anschließende Aminolyse mit Ethylendiamin erzielt wird. Die vorzugsweise ohne Lösungsmittel durchgeführte Aminolyse wird hierbei mit bis zu 500fachem Aminüberschuß pro Ester-Gruppierung bei Temperaturen von 0 bis ca 130 °C durchgeführt.

Als Beispiel einer Epoxid-Anlagerung sei die Umsetzung von 6,6',6",6"',6"",6""'-Hexaamino-6,6',6",6"',6"",6""'-hexadeoxy-α-cyclodextrin mit 1,3-[N,N'-Tetrabenzyl]-diamino-2-[oxiranylmethoxy]-propan und anschließende Freisetzung der Aminofunktionen durch katalytische Hydrierung nach den dem Fachmann bekannten Verfahren (s. auch oben) genannt.

Ein Teil der über die Komplexbildner-Einheiten K eingeführten Säuregruppen der so erhaltenen Polymer-Verbindungen kann gewünschtenfalls nach dem Fachmann bekannten Verfahren weiter funktionalisiert werden, zum Beispiel durch Überführung in Ester oder Amid-, Gruppen.

Die Herstellung der erfindungsgemäßen Metallkomplexe erfolgt in der Weise, wie sie in der Deutschen Offenlegungsschrift 34 01 052 offenbart worden ist, indem man das Metalloxid oder ein Metallsalz (beispielsweise das Nitrat, Acetat, Carbonat, Chlorid oder Sulfat) des Elements der Ordnungszahlen 21 - 29, 42, 44, 57 - 83 in Waser und/oder einem niederen Alkohol (wie Methanol, Ethanol oder Isopropanol) löst oder suspendiert und mit der Lösung oder Suspension der äquivalenten Menge des komplexbildenden Liganden umsetzt und anschließend, falls gewünscht, vorhandene acide Wasserstoffatome der Säure-bzw. Phenolgruppen durch Kationen anorganischer und/oder organischer Basen oder Aminosäuren substituiert.

Die Einführung der gewünschten Metallionen kann sowohl auf der Stufe der Komplexbildner, I'B, d.h. vor der Kopplung an die Kaskaden-Polymere, als auch nach Kopplung der unmetallierten Liganden, I'B erfolgen.

Die Neutralisation erfolgt dabei mit Hilfe anorganischer Basen (zum Beispiel Hydroxiden, Carbonaten oder Bicarbonaten) von zum Beispiel Natrium, Kalium, Lithium, Magnesium oder Calcium und/oder organischer Basen wie unter anderem primarer, sekundärer und tertiärer Amine, wie zum Beispiel Ethanolamin, Morpholin, Glucamin, N-Methyl- und N,N-Dimethylglucamin, sowie basischer Aminosäuren, wie zum Beispiel Lysin, Arginin und Ornithin oder von Amiden ursprünglich neutraler oder saurer Aminosäuren.

Zur Herstellung der neutralen Komplexverbindungen kann man beispielsweise den sauren Komplexsalzen in wäßriger Lösung oder Suspension soviel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits wahrend der Komplexbildung der Reaktionsmischung zuzusetzen und dadurch einen Verfahrensschritt einzusparen.

Enthalten die sauren Komplexverbindungen mehrere freie acide Gruppen, so ist es oft zweckmäßig, neutrale Mischsalze herzustellen, die sowohl anorganische als auch organische Kationen als Gegenionen enthalten.

Dies kann beispielsweise geschehen, indem man den komplexbildenden Liganden in wäßriger Suspension oder Losung mit dem Oxid oder Salz des das Zentralion liefernden Elements und der Hälfte der zur Neutralisation benötigten Menge einer organischen Base umsetzt, das gebildete Komplexsalz isoliert, es gewünschtenfalls reinigt und dann zur vollständigen Neutralisation mit der benötigten Menge anorganischer Base versetzt. Die Reihenfolge der Basenzugabe kann auch umgekehrt werden.

Eine andere Möglichkeit, zu neutralen Komplexverbindungen zu kommen, besteht darin, die verbleibenden Sauregruppen im Komplex ganz oder teilweise in zum Beispiel Ester oder Amide zu überführen. Dies kann durch nachträgliche Reaktion am fertigen Polymer-Komplex geschehen (z.B. durch erschöpfende Umsetzung der freien Carboxy-Gruppen mit Dimethylsulfat) wie auch durch Verwendung eines geeignet derivatisierten Substrats zur Einführung der Komplexbildner-Einheiten der allgemeinen Formeln I'B.

Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusatze - in wäßrigem Medium suspendiert oder löst und anschließend die Suspension oder Losung gegebenenfalls sterilisiert. Geeignete Zusätze sind beispielsweise physiologisch unbedenkliche Puffer (wie zum Beispiel Tromethamin), Zusatze von Komplexbildnern (wie zum Beispiel Diethylentriaminpentaessigsäure) oder - falls erforderlich - Elektrolyte wie zum Beispiel Natriumchlorid oder - falls erforderlich - Antioxidantien wie zum Beispiel Ascorbinsäure.

Sind für die enterale Verabreichung oder andere Zwecke Suspensionen oder Lösungen der erfindungsgemäßen Mittel in Wasser oder physiologischer Salzlosung erwünscht, werden sie mit einem oder mehreren in der Galenik üblichen Hilfsstoff(en) (zum Beispiel Methylcellulose, Lactose, Mannit) und/oder Tensid(en) (zum Beispiel Lecithine, Tween^{(R)}, Myrj^{(R)} und/oder Aromastoff(en) zur Geschmackskorrektur (zum Beispiel ätherischen Ölen) gemischt.

Prinzipiell ist es auch möglich, die erfindungsgemäßen pharmazeutischen Mittel auch ohne Isolierung der Komplexsalze herzustellen. In jedem Fall muß besondere Sorgfalt darauf verwendet werden, die Chelatbildung so vorzunehmen, daß die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Die erfindungsgemäßen pharmazeutischen Mittel enthalten vorzugsweise 1µMol -1 Mol/l des Komplexsalzes und werden in der Regel in Mengen von 0,0001 - 5 mMol/kg dosiert. Sie sind zur enteralen und parenteralen Applikation bestimmt. Die erfindungsgemäßen Komplexverbindungen kommen zur Anwendung
1. für die NMR- und Röntgen-Diagnostik in Form ihrer Komplexe mit den Ionen der Elemente mit den Ordnungszahlen 21 - 29, 39, 42, 44 und 57-83;
2. für die Radiodiagnostik und Radiotherapie in Form ihrer Komplexe mit den Radioisotopen der Elemente mit den Ordnungszahlen 27, 29, 31, 32, 37-39, 43, 49, 62. 64, 70, 75 und 77.

Die erfindungsgemäßen Mittel erfüllen die vielfältigen Voraussetzungen für die Eignung als Kontrastmittel für die Kernspintomographie. So sind sie hervorragend dazu geeignet, nach oraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten, und die gute Verträglichkeit, die notwendig ist, um den nichtinvasiven Charakter der Untersuchungen aufrechtzuerhalten.

Die gute Wasserlöslichkeit und geringe Osmolalität der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen, das heißt NMR-Diagnostika müssen 100 bis 1000fach besser wasserloslich sein als für die NMR-Spektroskopie. Weiterhin weisen die erfindungsgemaßen Mittel nicht nur eine hohe Stabilität in-vitro auf, sondern auch eine überraschend hohe Stabilität in-vivo, so daß eine Freigabe oder ein Austausch der in den Komplexen nicht kovalent gebundenen - an sich giftigen - Ionen innerhalb der Zeit, in der die neuen Kontrastmittel vollständig wieder ausgeschieden werden, nur äußerst langsam erfolgt.

Im allgemeinen werden die erfindungsgemäßen Mittel für die Anwendung als NMR-Diagnostika in Mengen von 0,0001 - 5 mMol/kg, vorzugsweise 0,005 - 0.5 mMol/ kg. dosiert. Details der Anwendung werden zum Beispiel in H.J. Weinmann et al., Am. J. of Roentgenology 142, 619 (1984) diskutiert.

Besonders niedrige Dosierungen (unter 1 mg/kg Körpergewicht) von organspezifischen NMR-Diagnostika sind zum Beispiel zum Nachweis von Tumoren und von Herzinfarkt einsetzbar.

Ferner können die erfindungsgemäßen Komplexverbindungen vorteilhaft als Suszeptibilitäts-Reagenzien und als shift-Reagenzien für die in-vivo-NMR-Spektroskopie verwendet werden.

Die erfindungsgemäßen Mittel sind aufgrund ihrer günstigen radioaktiven Eigenschaften und der guten Stabilität der in ihnen enthaltenen Komplexverbindungen auch als Radiodiagnostika geeignet. Details ihrer Anwendung und Dosierung werden z.B. in "Radiotracers for Medical Applications", CRC-Press, Boca Raton, Florida, beschrieben.

Eine weitere bildgebende Methode mit Radioisotopen ist die Positronen-Emissions-Tomographie, die positronenemittierende Isotope wie z.B. ⁴³Sc, ⁴⁴Sc, ⁵²Fe, ⁵⁵Co und ⁵⁸Ga verwendet [Heiss, W.D.; Phelps, M.E.; Positron Emission Tomography of Brain, Springer Verlag Berlin, Heidelberg, New York 1983).

Die erfindungsgemäßen Verbindungen können auch in der Radioimmuno- oder Strahlentherapie verwendet werden. Diese unterscheidet sich von der entsprechenden Diagnostik nur durch die Menge und Art des verwendeten Isotops. Ziel ist dabei die Zerstörung von Tumorzellen durch energiereiche kurzwellige Strahlung mit einer möglichst geringen Reichweite. Geeignete β-emittierende Ionen sind zum Beispiel ⁴⁶Sc, ⁴⁷Sc, ⁴⁸Sc, ⁷²Ga, ⁷³Ga und ⁹⁰Y. Geeignete geringe Halbwertzeiten aufweisende α-emittierende Ionen sind zum Beispiel ²¹¹Bi, ²¹²Bi, ²¹³Bi und ²¹⁴Bi, wobei ²¹²Bi bevorzugt ist. Ein geeignetes Photonen- und Elektronenemittierendes lon ist ¹⁵⁸Gd, das aus ¹⁵⁷Gd durch Neutroneneinfang erhalten werden kann.

Die erfindungsgemäßen therapeutischen Mittel werden parenteral, vorzugsweise i.v. appliziert.

Details der Anwendung von Radiotherapeutika werden z.B. in R.W. Kozak et al. TIBTEC, Oktober 1986, 262, diskutiert.

Die erfindungsgemaßen Mittel sind hervorragend als Röntgenkontrastmittel geeignet, wobei besonders hervorzuheben ist, daß sich mit ihnen keine Anzeichen der von den jodhaltigen Kontrastmitteln bekannten anaphylaxieartigen Reaktionen in biochemisch-pharmakologischen Untersuchungen erkennen lassen. Besonders wertvoll sind sie wegen der günstigen Absorptionseigenschaften in Bereichen höherer Rohrenspannungen für digitale Substraktionstechniken.

Im allgemeinen werden die erfindungsgemäßen Mittel für die Anwendung als Rontgenkontrastmittel in Analogie zu zum Beispiel Meglumin-Diatrizoat in Mengen von 0,1 - 5 mMol/kg, vorzugsweise 0,25 - 1 mMol/kg, dosiert.

Details der Anwendung von Röntgenkontrastmitteln werden zum Beispiel in Barke, Röntgenkontrastmittel, G. Thieme, Leipzig (1970) und P. Thurn. E. Bücheler - "Einführung in die Röntgendiagnostik", G. Thieme, Stuttgart, New York (1977) diskutiert.

Insgesamt ist es gelungen, neue Komplexbildner, Metallkomplexe und Metallkomplexsalze zu synthetisieren, die neue Möglichkeiten in der diagnostichen und therapeutischen Medizin erschließen. Vor allem die Entwickklung neuartiger bildgebender Verfahren in der medizinischen Diagnostik läßt diese Entwicklung wünschenswert erscheinen.

Die nachfolgenden Beispiele dienen zur näheren Erläuterung des Erfindungsgegenstands.

### Beispiel 1

a) **1,2-Epoxy-3-dibenzylaminopropan**
Zu einer gut gerührten Suspension aus 234.51 g (2,53 mol) Epichlorhydrin und 200 ml 32 % Natronlauge tropft man bei 0°C 100 g (506.9 mmol) Dibenzylamin (gelöst in 300 ml Methylenchlorid). Man rührt 2 Stunden bei 0°C dann 3 Stunden bei Raumtemperatur. Es wird mit 3 l Wasser verdünnt und 3 mal mit 500 ml Methylenchlorid extrahiert. Die organischen Phasen werden vereinigt, über Magnesiumsulfat getrocknet und im Vakuum eingedampft. Das zurückbleibende Öl wird an Kieselgel "Flash"-chromatographiert (Laufmittel: Methylenchlorid/Hexan/Aceton: 20/10/3).
Ausbeute: 111,72 g eines farblosen Öls (87 % der Theorie)

| Analyse: | | | |
|---|---|---|---|
| C 80,60 | H 7,56 | N 5,53 | (Ber.) |
| C 80,62 | H 7,50 | N 5,48 | (Gef.) |

b) **10-(3-Dibenzylamino-2-hydroxypropyl)-1,4,7-triscarboxylmethyl-1,4,7,10-tetraazacyclododecan**
20 g (78,95 mmol) der Titelverbindung aus Beispiel 1a) und 20,51 g (59,21 mmol) 1,4,7-Triscarboxymethyl-1,4,7,10-tetraazacyclododecan (≙ DO3A) werden in einer Mischung aus 50 ml Dioxan 200 ml Wasser gelöst, und der pH-Wert mit 6N Kalilauge auf pH 10 gebracht. Man rührt 24 Stunden bei 40°C.
Man dampft zur Trockene ein, nimmt den Rückstand mit 500 ml Wasser/500 ml Methanol auf und extrahiert 2 mal mit 200 ml tert-Buthyl-methylether Die wässrige Lösung wird mit 5N Salzsäure auf pH 3 gestellt und zu Trockene eingedampft. Der Rückstand wird im Vakuum eingeengt und anschließend auf eine Säule aus Poly-(4-vinylpyridin) geben. Das Produkt wird mit einer Lösung aus Ethanol/Wasser 1:1 eluiert. Nach Eindampfen im Vakuum erhält man 22,37 g (63 % der Theorie, bezogen auf DO3A) der Titelverbindung als stark hygroskopischen, glasigen Feststoff (6,9 % Wasser laut Analyse).

| Analyse: | | | |
|---|---|---|---|
| C 62,08 | H 7,56 | N 11,68 | (Ber.) |
| C 62,15 | H7,61 | N 11,61 | (Gef.) |

c) **Gadolinium Komplex Von 10-(3-Dibenzylamino-2-hydroxypropyl)-1,4,7-tris-carboxymethyl-1,4,7,10-tetraazacyclododecan**
21 g (35,02 mmol) der Titelverbindung aus Beispiel 1b) werden in einer Lösung aus 150 ml entionisiertem Wasser/50 ml Methanol gelöst und 6,35 g (17,51 mmol) Gadoliniumoxid zugesetzt. Man kocht 2 Stunden unter Rückfluß. Es werden 3 g Aktivkohle zugesetzt. Die Lösung wird heiß filtriert und das Filtrat im Vakuum zur Trockene eingedampft. Ausbeute 25,08 g (95 % der Theorie) eines glasigen Feststoffes (5,2 % Wasser laut Analyse).

| Analyse: | | | | |
|---|---|---|---|---|
| C 49,39 | H 5,61 | N 9,29 | Gd 20,86 | (Ber.) |
| C 49,41 | H 5,70 | N 9,25 | Gd 20,88 | (Gef.) |

d) **Gadolinium Komplex von 10-(3-Amino-2-hydroxypropyl)-1,4,7-triscarboxymethyl-1,4,7,10-tetraazacyclododecan**
24 g (31,83 mmol) der Titelverbindung aus Beispiel 1c) werden in einer Mischung aus 250 ml entionisiertem Wasser/150 ml Methanol gelöst und 10 g Palladiumkatalysator (10 % Pd auf Aktivkohle) zugesetzt. Anschließend wird 24 Stunden bei 50°C hydriert. Man filtriert vom Katalysator ab und dampft das Filtrat im Vakuum ein. Ausbeute: 17,89 g (98 % der Theorie) der Titelverbindung als glasigen Feststoff (6,4 % Wasser laut Analyse).

| Analyse: | | | | |
|---|---|---|---|---|
| C 35,59 | H 5,27 | N 12,21 | Gd 27,41 | (Ber.) |
| C 35,51 | H 5,34 | N 12,16 | Gd 27,36 | (Gef.) |

e) **Gadolinium Komplex von 10-(3-Isothiocyanato-2-hydroxypropyl)-1,4,7-triscarboxylmethyl-1,4,7,10-tetraazacyclododecan**
Zu einer Lösung aus 12 g (20.92 mmol) der Titelverbindung aus Beispiel 1d) in 500 ml entionisiertem Wasser und 20 ml Polyvinylpyridin (Reillex) gibt man eine Lösung aus 4,81 g (41,83 mmol) Thiophosgen in 100 ml Chloroform. Die zweiphasige Lösung wird 10 Minuten bei 40°C, dann eine Stunde bei Raumtemperatur gerührt und filtriert. Die organische Phase wird abgetrennt und die Wasserphase 2 mal mit 200 ml Chloroform nachextrahiert. Anschließend wird die Wasserphase gefriergetrocknet. Ausbeute: 12,62 g (98 % der Theorie) eines farblosen Pulvers (5,7 % Wasser laut Analyse).

| Analyse: | | | | | |
|---|---|---|---|---|---|
| C 35,11 | H 4,58 | N 11,37 | S 5,21 | Gd 25,54 | (Ber.) |
| C 35,04 | H 4,64 | N 11,31 | S 5,15 | Gd 25,48 | (Gef.) |

### Beispiel 2

a) **1-Dibenzylamino-5,6-epoxy-3-oxahexan**
100 g (414 mmol) N-Dibenzylaminoethanol werden in 200 ml Methylenchlorid gelöst und bei 0°C zu einer stark gerührten Mischung aus 250 ml 50 % Natronlauge, 7,03 g (20,7 mmol) Tetra-n-butylammoniumhydrogensülfat 153,4 g (1,657 mol) Epichlorhydrin getropft. Man rührt 8 Stunden bei 0° C, über Nacht bei Raumtemperatur. Man verdünnt mit 2 l Wasser und extrahiert 3 mal mit 500 ml Methylenchlorid Die vereinigten organischen Phasen werden über Magnesiumsulfat getrocknet und im Vakuum eingedampft. Das zurückbleibende Öl wird einer Flash-Chromatographie unterworfen. (Kieselgel/Laufmittel: Methylenchlorid/Hexan/Aceton 20/10/3)
Ausbeute: 96.12 g (78 % der Theorie) eines farblosen Öls.

| Analyse: | | | |
|---|---|---|---|
| C 76,74 | H 7,79 | N 4,71 | (Ber.) |
| C 76,68 | H 7,85 | N 4,66 | (Gef.) |

b) **10-(6-Dibenzylamino-2-hydroxy-4-oxahexyl)-1,4,7-triscarboxymethyl-1,4,7,10-tetraazacyclododecan**
34,34 g (115,47 mmol) der Titelverbindung aus Beispiel 2a) und 20 g (57,74 mmol) 1,4,7-Triscarboxymethyl-1,4,7,10-tetraazacyclododecan (≙ DO3A) werden in einer Mischung aus 60 ml Dioxan/350 ml Wasser gelöst und der pH-Wert mit 6N Kalilauge auf pH 10 gestellt. Man rührt 24 Stunden bei 40°C. Es wird wie unter Beispiel 1b) beschrieben aufgearbeitet.
Ausbeute: 26,39 g (71 % der Theorie bezogen auf DO3A) eines glasigen Fettstoffes (7,1 % Wasser laut Analyse).

| Analyse: | | | |
|---|---|---|---|
| C 61,57 | H 7,67 | N 10,88 | (Ber.) |
| C 61,49 | H 7,80 | N 10,79 | (Gef.) |

c) **Gadolinium Komplex von 10-(6-Dibenzylamino-2-hydroxy-4-oxahexyl)-1,4,7-triscarboxymethyl-1,4,7,10-tetraazacyclododecan**
23 g (35,73 mmol) der Titelverbindung aus Beispiel 2b) werden in einer Lösung aus 150 ml entionisiertem Wasser/50 ml Methanol gelöst und 6,48 g (17,86 mmol) Gadoliniumoxid zugesetzt. Man kocht 2 Stunden unter Rückfluß. Es werden 3 g Aktivkohle zugesetzt und eine zweite Stunde refluxiert. Die Lösung wird heiß filtriert und das Filtrat im Vakuum zur Trockene eingedampft. Man erhält 27,65 g (97 % der Theorie) der Titelverbindung als glasigen Feststoff (7,8 % Wasser laut Analyse).

| Analyse: | | | | |
|---|---|---|---|---|
| C 49,67 | H 5,81 | N 8,78 | Gd 19,71 | (Ber.) |
| C 49,61 | H 5,89 | N 8,71 | Gd 19,61 | (Gef.) |

d) **Gadolinium Komplex von 10-(6-Amino-2-hydroxy-4-oxahexyl)-1,4,7-triscarboxymethyl-1,4,7,10-tetraazacyclododecan**
25 g (31,33 mmol) der Titelverbindung aus Beispiel 2c) werden in einer Mischung aus 250 ml entionisiertem Wasser/150 ml Methanol gelöst und 10 g Palladiumkatalysator (10 % Pd auf Aktivkohle) zugesetzt. Anschließend wird 24 Stunden bei 50°C hydriert. Man filtriert vom Katalysator ab und dampft das Filtrat im Vakuum ein.
Ausbeute: 19,16 g (99 % der Theorie) der Titelverbindung als glasigen Feststoff (5,7 % Wasser laut Analyse).

| Analyse: | | | | |
|---|---|---|---|---|
| C 36,94 | H 5,55 | N 11,34 | Gd 25,45 | (Ber.) |
| C 36,88 | H 5,59 | N 11,27 | Gd 25,38 | (Gef.) |

e) **Gadolinium Komplex von 10-(6-Isothiocyanato-2-hydroxy-4-oxahexyl)-1,4,7-triscarboxymethyl-1,4,7,10-tetraazacyclododecan**
Zu einer Lösung aus 15 g (24,28 mmol) der Titelverbindung aus Beispiel 2d) in 500 ml entionisiertem Wasser und 20 ml Polyvinylpyridin (Reillex)) gibt man eine Lösung aus 5,58 g (48,56 mmol) Thiophosgen in 100 ml Chloroform. Die zweiphasige Lösung wird 10 Minuten bei 40°C, dann eine Stunde bei Raumtemperatur gerührt und filtriert. Die organische Phase wird abgetrennt und die Wasserphase 2 mal mit 200 ml Chloroform extrahiert. Anschließend wird die Wasserphase gefriergetrocknet.
Ausbeute: 15,7 g (98 % der Theorie) eines farblosen Pulvers (6,1 % Wasser laut Analyse).

| Analyse: | | | | | |
|---|---|---|---|---|---|
| C 36,41 | H 4,89 | N 10,61 | Gd 23,83 | S 4,86 | (Ber.) |
| C 36,35 | H 4,95 | N 10,51 | Gd 23,71 | S 4,78 | (Gef.) |

### Beispiel 3

### Gadolinium Komplex von 10-(9-Brom-2-hydroxy-8-oxo-4-oxa-7-azanonyl)-1,4,7-triscarboxymethyl-1,4,7,10-tetraazacyclododecan

10 g (16,19 mmol) der Titelverbindung aus Beispiel 2e) werden in 50 ml Wasser gelöst und mit 3N Natronlauge auf pH 9 gebracht. Hierzu wird bei 0°C eine Lösung von 4,25 g (21,04 mmol) Bromacetylbromid in 20 ml Dioxan zugetropft und der pH-Wert durch Zugabe von 3N Natronlauge bei pH 9 gehalten. Man rührt 1 Stunde bei 0°C. 2 Stunden bei Raumtemperatur. Es wird im Vakuum eingedampft und der Rückstand chromatographiert (Li Chroprep RP-18, Merck/Laufmittel: Acetonitril/H₂O - Gradient). Nach Eindampfen der Hauptfraktionen im Vakuum ergeben sich 10,64 g (89 % der Theorie) der Titelverbindung als kristalliner Feststoff (5,4 % Wasser laut Analyse).

| Analyse: | | | | | |
|---|---|---|---|---|---|
| C 34,15 | H 4,78 | N 9,48 | Gd 21,29 | Br 10,82 | (Ber.) |
| C 34,11 | H 4,85 | N 9,41 | Gd 21,19 | Br 10,75 | (Gef.) |

### Beispiel 4

a) **1-Dibenzylamino-5-hydroxy-3-oxapentan**
Eine Mischung aus 50 g (475,56 mmol) 2-(2-Amino-aethoxy) aethanol und 144,6 g (1,046 mol) Kaliumcarbonat in 600 ml EtOH/60 ml Wasser wird auf 60°C erhitzt. Zu dieser Mischung tropft man innerhalb 1 Stunde 178,95 g (1,046 mol) Benzylbromid zu und kocht anschließend 2 Stunden unter Rückfluß. Man dampft im Vakuum ein, nimmt den Rückstand mit 1 Methylenchlorid auf und filtriert von den Salzen ab. Das Filtrat wird im Vakuum eingeengt und durch Flash-Chromatographie gereinigt. (Kieselgel/Lautmittel: Methylenchlorid/Hexan/Aceton: 10/5/1)
Ausbeute: 127,58 g (94 % der Theorie) eines farblosen Öls.

| Analyse: | | | |
|---|---|---|---|
| C 75,76 | H 8,12 | N 4,91 | (Ber.) |
| C 75,71 | H 8,18 | N 4,85 | (Gef.) |

b) **1-Dibenzylamino-8,9-epoxy-3,6-dioxanonan**
Zu einer gut gerührten Suspension aus 162,11 g (1,752 mol) Epichlorhydrin, 8,2 g (24,15 mmol) Tetranbutylammoniumhydrogensulfat und 250 ml 50 % Natronlauge tropft man bei 0°C eine Lösung aus 125 g (438 mmol) der Titelverbindung aus Beispiel 4a) in 200 ml Methylenchlorid. Man rührt 8 Stunden bei 0°C, über Nacht bei Raumtemperatur. Es wird mit 2 l Wasser verdünnt und 2 mal mit 500 ml Methylenchlorid extrahiert. Die vereinigten organischen Phasen werden über Magnesiumsulfat getrocknet und im Vakuum eingedampft. Das zurückbleibende Öl wird durch Flash-Chromatographie gereinigt (Kieselgel/Laufmittel: Methylenchlorid/Hexan/ Aceton: 20/10/3)
Ausbeute: 116,5 g (78 % der Theorie) eines farblosen Öls.

| Analyse: | | | |
|---|---|---|---|
| C 73,87 | H 7,79 | N 4,10 | (Ber.) |
| C 73,78 | H 7,95 | N 4,03 | (Gef.) |

c) **10-(9-Dibenzylamino-2-hydroxy-4,7-dioxanonyl)-1,4,7-triscarboxymethyl-1,4,7,10-tetraazacyclododecan**
39,43 g (115,47 mmol) der Titelverbindung aus Beispiel 4b) und 20 g (57,74 mmol) 1,4,7-Triscarboxymethyl-1,4,7,10-tetraazacyclododecan (≙ DO3A) werden in einer Mischung aus 60 ml Dioxan/250 ml Wasser gelöst und der pH-Wert mit 6N Kalilauge auf pH 10 gestellt. Man rührt 24 Stunden bei 40°C. Anschließend wird, wie unter Beispiel 1b) beschrieben, aufgearbeitet.
Ausbeute: 28,59 g (72 % der Theorie bzg. auf D03A) eines glasigen Feststoffes (6,3 % Wasser laut Analyse).

| Analyse | | | |
|---|---|---|---|
| C 61,12 | H 7,77 | N 10,18 | (Ber.) |
| C 61,07 | H 7,84 | N 10,05 | (Gef.) |

d) **Gadolinium Komplex von 10-(9-Dibenzylamino-2-hydroxy-4,7-dioxanonyl)-1,4,7-triscarboxymethyl-1,4,7,10-tetraazacyclododecan**
25 g (36,35 mmol) der Titelverbindung aus Beispiel 4c) werden in einer Lösung aus 150 ml entionisiertem Wasser/50 ml Methanol gelöst und 6,59 g (18,17 mmol) Gadoliniumoxid zugesetzt. Man kocht 2 Stunden unter Rückfluß. Es werden 3 g Aktivkohle zugesetzt und eine zweite Stunde refluxiert. Die Lösung wird heiß filtriert und das Filtrat im Vakuum zur Trockene eingedampft.
Ausbeute: 30,0 g (98 % der Theorie) der Titelverbindung als glasiger Feststoff (5,4 % Wasser laut Analyse).

| Analyse: | | | | |
|---|---|---|---|---|
| C 49,92 | H 5,98 | N 8,32 | Gd 18,67 | (Ber.) |
| C 49,83 | H 5,90 | N 8,34 | Gd 18,58 | (Gef.) |

In analoger Weise erhält man mit Eu ¹⁵¹Eu₂O₃ den entsprechenden Europium-Komplex.

| Analyse: | | | | |
|---|---|---|---|---|
| C 50,24 | H 6,02 | N 8,37 | Eu 18,16 | (Ber.) |
| C 50,17 | H 5,96 | N 8,29 | Eu 18,09 | (Gef.) |

e) **Gadolinium Komplex von 10-(9-Amino-2-hydroxy-4,7-dioxanonyl)-1,4,7-triscarboxymethyl-1,4,7,10-tetraazacyclododecan**
29 g (34,44 mmol) der Titelverbindung aus Beispiel 4d) werden in einer Mischung aus 250 ml entionisiertem Wasser/150 ml Methanol gelöst und 10 g Palladiumkatalysator (10 % Pd auf Aktivkohle) zugesetzt. Anschließend wird 24 Stunden bei 50°C hydriert. Man filtriert vom Katalysator ab und dampft das Filtrat im Vakuum ein.
Ausbeute: 22,56 g (99 % der Theorie) der Titelverbindung als glasigen Feststoff (6,5 % Wasser laut Analyse)

| Analyse: | | | | |
|---|---|---|---|---|
| C 38,11 | H 5,79 | N 10,58 | Gd 23,76 | (Ber.) |
| C 38,05 | H 5,86 | N 10,47 | Gd 23,65 | (Gef.) |

f) **Gadolinium-Komplex von 10-(9-Isothiocyanato-2-hydroxy-4,7-dioxanonyl)-1,4,7-triscarboxymethyl-1,4,7,10-tetraazacyclododecan**
Zu einer Lösung aus 15 g (22,66 mmol) der Titelverbindung aus Beispiel 4e) in 500 ml entionisiertem Wasser und 20 ml Polyvinylpyridin (Reillex) gibt man eine Lösung aus 5,21 g (45,33 mmol) Thiophosgen in 100 ml Chloroform. Die zweiphasige Lösung wird 10 Minuten bei 40°C, dann eine Stunde bei Raumtemperatur gerührt und filtriert. Die organische Phase wird abgetrennt und die Wasserphase 2 mal mit 200 ml Chloroform extrahiert. Anschließend wird die Wasserphase gefriergetrocknet. Ausbeute: 15,64 g (98 % der Theorie) eines farblosen Pulvers (5,9 % Wasser laut Analyse)

| Analyse: | | | | | |
|---|---|---|---|---|---|
| C 37,54 | H 5,15 | N 9,95 | Gd 22,34 | S 4,55 | (Ber.) |
| C 37,49 | H 5,11 | N 9,91 | Gd 22,27 | S 4,61 | (Gef.) |

### Beispiel 5

a) **Hexa-methylester des Tris(aminoethyl)amin-Kaskadenpolymers**
7,6 ml Tris(aminoethyl)amin (50 mmol) werden in 10 ml Methanol gelöst und tropfenweise zu 54,5 ml (600 mmol) Methylacrylat gegeben. Die Mischung wird 3 Tage bei Raumtemperatur gerührt und anschließend im Vakuum eingedampft. Das verbleibende Öl wird aus Methanol/Ether/Hexan umgefällt.
Ausbeute: 30,59 g (92,3 % der Theorie) leicht gelbliches Öl.

| Analyse: | | | |
|---|---|---|---|
| C 54,37 | H 8,21 | N 8,45 | (Ber.) |
| C 54,32 | H 8,29 | N 8,43 | (Gef.) |

b) **Hexa-Amin des Tris(aminoethyl)amin-Kaskadenpolymers**
26,5 g des in Beispiel 5a) beschriebenen Hexa-methylesters (40 mmol) werden in 20 ml Methanol gelöst und langsam zu 242 ml Ethylendiamin (3,6 mol) getropft und anschließend 3 Tage bei Raumtemperatur gerührt. Die Lösung wird im Vakuum eingedampft und das verbleibende Öl aus Methanol/Ether umgefällt.
Ausbeute: 31,25 g (94 % der Theorie) leicht gelb gefärbtes Öl.

| Analyse: | | | |
|---|---|---|---|
| C 52,03 | H 9,46 | N 26,96 | (Ber.) |
| C 51,97 | H 9,49 | N 26,89 | (Gef.) |

c) **Dodeka-methylester des Tris(aminoethyl)amin-Kaskadenpolymers**
Zu 103 ml (1,14 mol) Methylacrylat werden 30,1 g 36,2 mmol) des in Beispiel 5b) beschriebenen Hexa-Amins in 50 ml Methanol so langsam zugetropft, daß die Lösung klar bleibt und 5 Tage bei Raumtemperatur gerührt. Nach Einengen im Vakuum wird mehrmals aus Methanol/Ether/Hexan umgefällt.
Ausbeute: 64,2 g (95,1 %) gelbliches Öl.
Im FAB-Massenspektrum ist der M+H⁺-Peak deutlich erkennbar. Eine analytische Probe zeigte nach Korrektur des gaschromatographisch bestimmten Methanols folgende Elementaranalyse:

| | | | |
|---|---|---|---|
| C 54,12 | H 8,11 | N 12,02 | (Ber.) |
| C 54,01 | H 8,19 | N 11,98 | (Gef.) |

d) **Dodeka-amin des Tris(aminoethyl)amin-Kaskadenpolymers**
64,0 g (34,3 mmol) des in Beispiel 5c) beschriebenen Dodeka-Methylesters werden in 50 ml Methanol gelöst und langsam zu 870 ml Ethylendiamin (13 mol) getropft und 5 Tage bei Raumtemperatur gerührt. Nach Einengen im Vakuum wird mehrmals aus Methanol/Ether umgefällt, bis dünnschichtchromatographisch kein Ethylendiamin mehr nachweisbar ist. Ausbeute: 73,7 g (97 %) zähes, gelbliches Öl. Im FAB-Massenspektrum ist der Quasimolekülpeak bei 2201 deutlich erkennbar. Eine analytische Probe zeigte nach Korrektur des gaschromatographisch bestimmten Methanols folgende Elementaranalyse:

| | | | |
|---|---|---|---|
| C 52,39 | H 9,07 | N 25,46 | (Ber.) |
| C 52,29 | H 9,21 | N 25,71 | (Gef.) |

e) **24-Methylester des Tris(aminoethyl)amin-Kaskadenpolymers**
68,0 g (30 mmol) des 12-Amins (Beispiel 5d) werden in 120 ml Methanol gelöst und so langsam zu 270 ml (3 mol) Methylacrylat zugetropft, daß die Lösung homogen bleibt (Zugabe innerhalb 3 Stunden). Nach 5 Tagen wird analog Beispiel 5c) aufgearbeitet.
Ausbeute: 119,7 g (93,5 %) gelbliches Öl. Das FAB-Massenspektrum zeigt das Quasimolekülion bei m/e=4268.
Eine analytische Probe zeigte nach Korrektur des gaschromatographisch bestimmten Methanols folgende Elementaranalyse:

| | | | |
|---|---|---|---|
| C 54,04 | H 8,08 | N 13,13 | (Ber.) |
| C 54,28 | H 8,01 | N 12,99 | (Gef.) |

f) **24-Amin des Tris(aminoethyl)amin-Kaskadenpolymers**
39,87 g (9,3 mmol) des 24-Methylesters werden in 100 ml Methanol gelöst und tropfenweise zu 1,5 l Ethylendiamin (23 mol) gegeben und nach 7 Tagen analog Beispiel 5d) aufgearbeitet. Man erhält 44,0 g (95,9 %) eines zähen, gelbfarbenen Öls. Die Substanz ist im HPLC-Chromatogramm in 1M NaClO₄ an Lichrospher DIOL 100, 500, 1000 (Merck) einheitlich.

| Analyse: | | | |
|---|---|---|---|
| C 52,51 | H 8,94 | N 24,95 | (Ber.) |
| C 52,17 | H 8,72 | N 25,27 | (Gef.) |

### Beispiel 6

a) **Penta-methylester des Diethylentriamin-Kaskadenpolymers**
5,54 ml Diethylentriamin (50 mmol) werden in 20 ml Methanol gelöst und tropfenweise zu 45,4 ml Methylacrylat (500 mmol) gegeben. Die Mischung wird 5 Tage bei Raumtemperatur gerührt und anschließend im Vakuum eingedampft. Das verbleibende Öl wird aus Methanol/Ether/Hexan umgefällt.
Ausbeute: 24,8 g (92,9 %) leicht gelbliches Öl

| Analyse: | | | |
|---|---|---|---|
| C 54,02 | H 8,12 | N 7,87 | (Ber.) |
| C 53,92 | H 8,06 | N 7,92 | (Gef.) |

b) **Penta-amin des Diethylentriamin-Kaskadenpolymers**
21,3 g des in Beispiel 6a) beschriebenen Penta-methylesters (40 mmol) werden in 20 ml Methanol gelöst und langsam zu 202 ml Ethylendiamin (3,0 mol) getropft und anschließend 3 Tage bei Raumtemperatur gerührt. Die Lösung wird im Vakuum eingedampft und das verbleibende Öl aus Methanol/Ether umgefällt.
Ausbeute: 24,8 g (92 %) leicht gelb gefärbtes Öl.

| Analyse: | | | |
|---|---|---|---|
| C 51,69 | H 9,42 | N 27,02 | (Ber.) |
| C 51,48 | H 9,36 | N 27,15 | (Gef.) |

c) **Deka-methylester des Diethylentriamin-Kaskadenpolymers**
Zu 68 ml (0,75 mol) Methylacrylat werden 20,7 g des in Beispiel 6b) beschriebenen Penta-Amins in 35 ml Methanol so langsam zugetropft, daß die Lösung klar bleibt und 5 Tage bei Raumtemperatur gerührt. Nach Einengen im Vakuum wird mehrmals aus Methanol Ether/Hexan umgefällt. Ausbeute: 39,8 g (84 %) gelbliches Öl. Im FAB-Massenspektrum ist der M+H⁺-Peak deutlich erkennbar. Nach Korrektur des gaschromatographisch bestimmten Methanol-Gehalts zeigte eine analytische Probe folgende Elementaranalyse:

| | | |
|---|---|---|
| C 54,00 | H 8,08 | N 11,86 |
| C 54,27 | H 8,16 | N 11,63 |

d) **Deka-amin des Diethylentriamin-Kaskadenpolymers**
39,5 g (25,7 mmol) des in Beispiel 6c) beschriebenen Deka-Methylesters werden in 30 ml Methanol gelöst und langsam zu 520 ml (7,8 mol) Ethylendiamin gegeben und 5 Tage bei Raumperatur gerührt. Nach Einengen im Vakuum wird mehrmals aus Methanol Ether umgefällt, bis dünnschichtchromatographisch kein Ethylendiamin mehr nachweisbar ist. Ausbeute: 44,9 g (96,2 %) gelbliches Öl. Im FAB-Massenspektrum ist der Quasimolekülpeak bei m e = 1815 gut zu erkennen. Nach Korrektur des gas-chromatographisch bestimmten Methanol-Gehalts zeigte eine analytische Probe folgende Elementaranalyse:

| | | | |
|---|---|---|---|
| C 52,27 | H 9,05 | N 25,46 | (Ber.) |
| C 52,11 | H 9,09 | N 25,67 | (Gef.) |

e) **20-Methylester des Diethylentriamin-Kaskadenpolymers**
41,8 g (23 mmol) des Deka-Amins (Beispiel 6d) werden in 100 ml Methanol gelöst und so langsam zu 200 ml (2,2 mol) Methylacrylat zugetropft, daß die Lösung homogen bleibt (2 Stunden). Nach 5 Tagen wird analog Beispiel 6c) aufgearbeitet.
Ausbeute: 72,0 g (88,5 %) gelbliches Öl.
Das FAB-Massenspektrum zeigt das Quasimolekülion bei m/e = 3536. Nach Korrektur des gas-chromatographisch bestimmten Methanol-Gehalts zeigte eine analytische Probe folgende Elementaranalyse:

| | | | |
|---|---|---|---|
| C 53,99 | H 8,06 | N 13,07 | (Ber.) |
| C 53,71 | H 8,14 | N 13,10 | (Gef.) |

f) **20-Amin des Diethylentriamin-Kaskadenpolymers**
68,7 g (19,4 mmol) des 20-Methylesters werden in 100 ml Methanol gelöst und tropfenweise zu 1,5 l (23 mol) Ethylendiamin gegeben und nach 7 Tagen analog Beispiel 6d) aufgearbeitet. Man erhält 74,3 g (93,5 %) eines zähen Öls. Das Öl ist HPLC-einheitlich in 1M NaClO₄ an Lichrospher DIOL-100, 500, 1000 (Merck). Die Titration einer analytichen Probe mit 1N HCl ergibt 97,8 % der Theorie.

| Analyse: | | | |
|---|---|---|---|
| C 52,46 | H 8,93 | N 24,95 | (Ber.) |
| C 51,98 | H 8,99 | N 24,49 | (Gef.) |

g) **40-Methylester des Diethylentriamin-Kaskadenpolymers**
20,5 g (5 mmol) des in Beispiel 9f) beschriebenen 20-Amins werden in 100 ml Methanol gelöst und innerhalb 5 Stunden zu 200 ml (2,2 mol) Methylacrylat bei 50°C zugetropft und 3 Tage bei dieser Temperatur gerührt. Nach mehrmaligem Umfällen aus Methanol/Ether/Hexan erhält man 34,3 g (91 %) zähes Öl.

| Analyse: | | | |
|---|---|---|---|
| C 53,99 | H 8,06 | N 13,56 | (Ber.) |
| C 53,69 | H 8,12 | N 13.21 | (Gef.) |

h) **40-Amin des Diethylentriamin-Kaskadenpolymers**
26,4 g (3,5 mmol) des im vorstehenden Beispiel 6g) erhaltenen 40-Esters werden in 100 ml Methanol gelöst und zu 1170 ml (17,5 mol) Ethylendiamin zugetropft und nach 7 Tagen analog Beispiel 6d) aufgearbeitet.
Ausbeute: 28,7 g (94,6 %) zähes, gelbliches Öl.
Das Öl ist HPLC-einheitlich in 1M NaClO₄ an Lichrospher DIOL-100, 500, 1000 (Merck). Die Titration einer analytischen Probe mit 1N HCl ergibt 95,3 % der Theorie.

| Analyse: | | | |
|---|---|---|---|
| C 52,54 | H 8,88 | N 24,73 | (Ber.) |
| C 52,73 | H 8,57 | N 24,35 | (Gef.) |

i) **Thio-ureido-Konjugat aus dem Gd-Komplex des 10-(6-isothiocyanato-2-hydroxy-4-oxa-hexyl)-1,4,7-triscarboxymethyl-1,4,7,10-tetraazacyclododecan mit dem 40-Amin des Diethylentriamin-Kaskadenpolymers**
2,17 g (0,25 mmol) des in Beispiel 6h) beschriebenen 40-Amins werden in 250 ml H₂O gelöst. Dazu werden unter Stickstoff portionsweise in fester Form 8,43 g (12 mmol; 1,2facher Überschuß) des in Beispiel 2e) beschriebenen Isothiocyanat-Gd-Komplexes gegeben und über Nacht bei Raumtemperatur gerührt. Nach Ultrafiltration (AMICON^{R} YM-10-Membran) wird die Leitfähigkeit der Lösung mittels Ionenaustauscher (Amberlite IR 120. H⁺-Form, und IRA 410, OH⁻-Form) auf ein Minimum eingestellt. Es wird vom Austauscher abfiltriert und gefriergetrocknet.
Ausbeute: 7,6 g (87 %)
H₂O-Gehalt: 6,3 %
Gd-Bestimmung (AAS): 15,6 %
T₁-Relaxivität (H₂O): 12,43 ± 0,51[l/mmol.sec]
(Plasma): 13,19 ± 0,42[l/mmol·sec]

| Analyse (wasserfrei): | | | | | |
|---|---|---|---|---|---|
| C 40,39 | H 5,87 | N 14,10 | Gd 17,94 | S 3,66 | (Ber.) |
| C 40,67 | H 6,15 | N 13,88 | Gd 16,88 | S 3,47 | (Gef.) |

In analoger Weise werden die folgenden Thio-ureido-Konjugate erhalten:
aus dem in Beispiel 1e) beschriebenen Isothiocyanat:

| | | | | | |
|---|---|---|---|---|---|
| C 39,65 | H 5,70 | N 14,85 | S 3,85 | Gd 18,89 | (Ber.) |
| C 40,12 | H 5,55 | N 14,31 | S 3,39 | Gd 18,71 | (Gef.) |

aus dem Beispiel 4f) beschriebenen Isothiocyanat:

| | | | | | |
|---|---|---|---|---|---|
| C 41,07 | H 6,03 | N 13,43 | S 3,48 | Gd 17,08 | (Ber.) |
| C 40,69 | H 5,97 | N 13,57 | S 3,61 | Gd 16,88 | (Gef.) |

### Beispiel 7

a) **10-[2,6,7,-Trihydroxy-4-oxa-heptyl]-1,4,7-tris-carboxymethyl-1,4,7,10-tetraazacyclododecan**
19,56 g (103,92 mmol) 2,2-Dimethyl-4-(2',3'-epoxy)-propoxy-methyl-1,3-dioxolan und 10 g (28,86 mmol) 1,4,7-Triscarboxymethyl-1,4,7,10-tetraazacyclododecan (=DO3A) werden in einer Mischung aus 50 ml Dioxan/80 ml Wasser gelöst, und der pH-Wert mit 6N Kalilauge auf pH 10 gebracht. Man rührt 24 Stunden bei 70°C. Man dampft zur Trockene ein, nimmt den Rückstand mit 200 ml Wasser/50 ml Methanol auf und extrahiert 2 mal mit 100 ml tert.-Butyl-methylether. Die wäßrige Lösung wird mit 5N-Salzsäure auf pH 3 gestellt und zur Trockene eingedampft. Der Rückstand wird mit 200 ml Methanol/80 ml Dichlormethan ausgekocht (extrahiert). Man kühlt im Eisbad ab und filtriert vom ausgefallenen Kaliumchlorid ab. Das Fittrat wird im Vakuum eingedampft, der Rückstand in 45 ml Wasser/20 ml Ethanol gelöst und anschließend auf eine Säule aus Poly-(4-vinylpyridin) gegeben. Das Produkt wird mit einer Lösung aus Ethanol/Wasser 1:3 eluiert. Nach Eindampfen im Vakuum wird der Rückstand an einer Reversed Phase-Säule (RP 18/Laufmittel= Gradient aus Wasser/Tetrahydrofuran) chromatographiert. Nach Eindampfen der Hauptfraktion erhält man 10,13 g (71 % d. Th.) eines stark hygroskopischen, glasigen Feststoffes.

| Analyse (bezogen auf wasserfreie Substanz): | | | |
|---|---|---|---|
| C 48,57 | H 7,74 | N 11,33 | ber. |
| C 48,46 | H 7,81 | N 11,24 | |

b) **Gd-Komplex des 10-(2,6,7-Trihydroxy-4-oxa-heptyl)-1,4,7-tris-carboxymethyl-1,4,7,10-tetraazacyclododecans**
8,56 g (17,3 mmol) der Titelverbindung aus Beispiel 7a werden in 50 ml entionisiertem Wasser gelöst und 3,13 g (8,65 mmol) Gadoliniumoxid zugesetzt. Es wird 3 Stunden auf 90°C erhitzt. Die abgekühlte Lösung wird eine Stunde mit 3 ml saurem lonenaustauscher (AMB 252c) und 3 ml schwach basischem Austauscher (IRA 67) gerührt. Es wird vom Austauscher abfiltriert und das Filtrat gefriergetrocknet.
Ausbeute: 11,0 g (98 % d. Th.) eines farblosen amorphen Pulvers

| Analyse (bezogen auf wasserfreie Substanz): | | | | |
|---|---|---|---|---|
| C 37,03 | H 5,44 | N 8,64 | Gd 24,24 | ber. |
| C 37,00 | H 5,51 | N 8,57 | Gd 24,18 | |

c) **Gd-Komplex des N-(5-Hydroxy-3-oxa-hexyl-DO3A)-48-Amino-Kaskadenpolymers**
38,93 g (60 mmol) des Gd-Komplexes aus Beispiel 7b werden in 400 ml Methanol gelöst, mit 25,67 g (120 mmol) NalO₄ versetzt und 4 Stunden unter Lichtausschluß verrührt. Dann wird vom Ungelösten abfiltriert und das Filtrat gefriergetrocknet. Das Lyophilisat wird mit 6,52 g (0,625 mmol = 30 mmol-NH₂) eines 48-Kaskadenamins in 750 ml Puffer pH 9,0 (Riedel de Haën, Borax/HCl) gelöst und nach Zugabe von 11,32 g (180 mmol) Natriumcyanoborhydrid 6 Tage bei Raumtemperatur gerührt. Die Lösung wird anschließend über eine Amicon-Ultrafiltrationsmembran YM5 entsalzt und schließlich gefriergetrocknet.
Ausbeute: 16,45 g (61 % d. Th.)
H₂O-Gehalt (Karl-Fischer): 9,8 %
Gd-Bestimmung (AAS): 15,75 %
T1-Relaxivität (H₂O): 12,35 ± 0,14 l/mmol·sec
(Plasma): 14,74 ± 0,33 l/mmol·sec

### Beispiel 8

### Gd-Komplex des N-(2-Carboxyethyl)-N-(5-hydroxy-3-oxa-hexyl-DO3A)-48-Amino-Kaskadenpolymers

2,2 g des in Beispiel 7c beschriebenen Polymers mit sekundärem Amin in den Verknüpfungen zwischen Komplex und backbone werden in 25 ml Methanol gelöst und zu einer Mischung aus 20 ml (220 mmol) Methylacrylat und 20 ml Methanol zugetropft und 3 Tage bei Raumtemperatur gerührt. Die Lösung wird im Vakuum eingedampft, das blaßgelbe Öl in 20 ml 1N NaOH gelöst und 3 Stunden bei Raumtemperatur verseift. Anschließend wird mit verd. HCI neutralisiert und die Lösung über eine Amicon-Ultrafiltrationsmembran YM5 entsalzt und schließlich gefriergetrocknet.
Ausbeute: 2,20 g
H₂O-Gehalt (Karl Fischer): 7,7 %
Gd-Bestimmung (AAS): 14,93 %
T₁-Relaxivität (H₂O): 11,47 ± 0,14 l/mmol·sec
(Plasma): 13,38 ± 0,07 l/mmol·sec
Die Papier-Elektropnorese des Polymers bei pH 9,0 (0,05 M Borax) und 10 V/cm zeigt eine Wanderung zur Anode, während die Ausgangsverbindung (Beispiel 7c) unter den gleichen Bedingungen zur Kathode wandert.

### Beispiel 9

### Gd-Komplex des N-(1,2-Dicarboxyethyl)-N-(5-hydroxy-3-oxa-hexyl-DO3A)-48-Amino-Kaskadenpolymers

Zu 14,3 g (110 mmol) Maleinsäure-monomethylester (Tokyo Chemical Industry Co. Ltd.) in 15 ml Methanol werden unter Eiskühlung 23 ml Triethylamin zugetropft. Man läßt auf Raumtemperatur erwärmen, gibt zu dieser Lösung 2,20 g des in Beispiel 7c beschriebenen Polymers in 25 ml Methanol tropfenweise zu und rührt die Mischung 3 Tage bei Raumtemperatur. Dann wird mit Diethylether versetzt, vom abgeschiedenen Öl abdekantiert, der verbleibende Rückstand in 20 ml 1N NaOH gelöst und 3 Stunden bei Raumtemperatur verseift. Anschließend wird mit verd. HCl neutralisiert und die Lösung über eine Amicon-Ultrafiltrationsmembran YM5 entsatzt und schließlich gefriergetrocknet.
Ausbeute: 2,13 g
H₂O-Gehalt (Karl-Fischer): 7,9 %
Gd-Bestimmung (AAS): 14,53 %
T₁ -Relaxivität (H₂O): 11,93 ± 0,27 l/mmol·sec
(Plasma): 13,27 ± 0,09 l/mmol·sec

### Beispiel 10

### Gd-Komplex des N-(Carboxymethyl)-N-(5-hydroxy-3-oxa-hexyl-DO3A)-48-Amino-Kaskadenpolymers

2,20 g des in Beispiel 7c beschriebenen Polymers werden in 25 ml H₂O gelöst und durch Zugabe von 1N NaOH auf pH 10 eingestellt. Dazu wird bei 50°C langsam eine Lösung 2,6 g (22 mmol) Natriumchloracetat in 20 ml H₂O zugetropft und durch Zugabe von 1N NaOH der pH-Wert bei 10 gehalten. Nach beendeter Zugabe wird über Nacht bei dieser Temperatur gerührt, anschließend mit verd. Salzsäure neutralisiert und die Lösung über eine Amicon-Ultrafiltrationsmembran YM5 entsalzt. Nach Gefriertrocknung erhält man 2,3 g flockiges Pulver.
H₂O-Gehalt (Karl-Fischer): 10,5 %
Gd-Bestimmung (AAS): 15,12 %
T₁-Relaxivität (H₂O): 12,25 ± 0,37 l/mmol·sec
(Plasma): 12,93 ± 0,14 l/mmol·sec

### Beispiel 11

### Gd-Komplex des N-(Carboxymethoxyacetyl)-N-(5-hydroxy-3-oxa-hexyl-DO3A)-48-Amino-Kaskadenpolymers

2,20 g des in Beispiel 7c beschriebenen Polymers werden in 25 ml H₂O gelöst und durch Zugabe von 1N NaOH auf pH 9 eingestellt. Dazu werden 850 mg (6,6 mmol) Diglycolsäure-anhydrid (Fluka) portionsweise unter Rühren zugegeben, wobei der pH-Wert durch Zugabe von 2N NaOH auf pH 9 gehalten wird. Nach beendeter Zugabe wird 15 Minuten nachgerührt, mit verd. Salzsäure neutralisiert, ultrafiltriert (Amicon YM5) und schließlich gefriergetrocknet.
Ausbeute: 2,43 g
H₂O-Gehalt (Karl-Fischer): 8,3 %
Gd-Bestimmung (AAS): 14,82 %
T1-Relaxivität (H₂O): 11,45 ± 0,23 l/mmol·sec
(Plasma): 13,74 ± 0,20 l/mmol·sec

### Beispiel 12

### Thio-ureido-Konjugat aus dem Gd-Komplex des 10-(6-Isothiocyanato-2-hydroxy-4-oxa-hexyl)-1,4,7-tris-carboxymethyl-1,4,7,10,-tetraazacyclodoecans mit dem Gd-Komplex des N-(5-Hydroxy-3-oxahexyl-DO3A)-48-Amino-Kaskadenpolymer

2,20 g des in Beispiel 7c beschriebenen Polymers werden in 25 ml H₂O gelöst. Dazu werden unter Stickstoff portionsweise in fester Form 3,09 g (4,7 mmol) des in Beispiel 2e beschriebenen Isothiocyanat-Gd-Komplexes gegeben und über Nacht bei Raumtemperatur gerührt. Nach Ultrafiltration (Amicon YM-10-Membran) wird die Leitfähigkeit der Lösung mittels lonenaustauscher (Amberlite IR 120, H⁺-Form und IRA-410, OH⁻-Form) auf ein Minimum eingestellt. Es wird vom Austauscher abfiltriert und gefriergetrocknet.
Ausbeute: 3,31 g
H₂O-Gehalt (Karl-Fischer): 7,3 %
Gd-Bestimmung (AAS): 15,32 %
T₁-Relaxivität (H₂O): 12,79 ± 0,30 l/mmol·sec
(Plasma): 14,21 ± 0,05 l/mmol·sec

### Beispiel 13

a) **10-(2,3,4-Trihydroxybutyl)-1,4,7-triscarboxymethyl-1,4,7,10-tetraazacyclododecan**
10,0 g (28,87 mmol) 1,4,7-Triscarboxymethyl-1,4,7-10-tetraazacyclododecan (DO3A) werden in 40 ml Wasser gelöst und der pH mit 5 normaler Natronlauge auf 13 eingestellt. Man fügt eine Lösung aus 6,24 g (43,30 mmol) 2-(2,2-Dimethyl-1,3-dioxolan-4-yl)-ethylenoxid (DE 3 150 917) in 10 ml Dioxan zu und rührt 24 Stunden bei Raumtemperatur. Es wird mit 60 ml Wasser verdünnt und dreimal mit 50 ml Ether extrahiert. Die wäßrige Phase wird mit 10 %iger Salzsäure auf pH 2 gebracht und eingedampft. Der Rückstand wird in etwas Wasser gelöst und auf eine Kationenaustauschersäule (IR 120) gegeben. Nach Spülung mit Wasser wird der Ligand mit 0,5 normaler wäßriger Ammoniak-Lösung eluiert. Die Fraktionen werden eingedampft, das Ammoniumsalz mit wenig Wasser aufgenommen und über eine Anionenaustauschersäule (IRA 67) gegeben. Man wäscht zuerst mit Wasser und eluiert dann mit 0,5 normaler wäßriger Ameisensäure. Man dampft im Vakuum ein, löst den Rückstand in wenig heißem Methanol und gibt Aceton hinzu, wobei die Titelverbindung auskristallisiert.
Ausbeute: 11,31 g (87 % d. Th.) eines weißen hygroskopischen Pulvers
H₂O-Gehalt (Karl-Fischer): 11,1 %

| Analyse (bezogen auf wasserfreie Substanz): | | | |
|---|---|---|---|
| C 47,99 | H 7,61 | N 12,44 | ber. |
| C 47,93 | H 7,67 | N 12,40 | |

b) **Gadolinium-Komplex des 10-(2,3,4-Trihydroxybutyl)-1,4,7-triscarboxymethyl-1,4,7,10-tetraazacyclododecans**
10,0 g (22,2 mmol) der nach Beispiel 13a erhaltenen Verbindung werden in 60 ml entionisiertem Wasser gelöst und 4,02 g (11,1 mmol) Gadoliniumoxid zugesetzt. Man erwärmt 3 Stunden auf 90°C. Die abgekühlte Lösung wird mit je 2 ml saurem lonenaustauscher (IR 120) und 2 ml basischem Austauscher (IRA 410) 1 Stunde bei Raumtemperatur gerührt. Man filtriert vom Austauscher ab und kocht das Filtrat mit Aktivkohle kurz auf.
Nach Filtrieren und Gefriertrocknung erhält man ein weißes, amorphes Pulver.
Ausbeute: 12,76 g (95 % d. Th.)
H₂O-Gehalt (Karl-Fischer): 12,3 %

| Analyse (bezogen auf wasserfreie Substanz): | | | | |
|---|---|---|---|---|
| C 35,73 | H 5,17 | Gd 25,99 | N 9,26 | ber. |
| C 35,68 | H 5,24 | Gd 25,93 | N 9,21 | |

c) **Gd-Komplex des N-(2-Hydroxy-propyl-DO3A)-48-Amino-Kaskadenpolymers**
13,8 g (20 mmol) des Gd-Komplexes aus Beispiel 13b werden in 120 ml Methanol gelöst, mit 8,56 g (40 mmol) NalO₄ versetzt und 4 Stunden unter Lichtausschluß verrührt. Dann wird vom Ungelösten abfiltriert und das Filtrat gefriergetrocknet. Das Lyophilisat wird mit 2,17 g (0,208 mmol = 10 mmol -NH₂) des in Beispiel 7c verwendeten 48-Kaskadenamins in 250 ml Puffer pH 9,0 (Riedel de Haën, Borax/HCl) gelöst und nach Zugabe von 3,77 g (60 mmol) Natriumcyanoborhydrid 6 Tage bei Raumtemperatur gerührt. Die Lösung wird anschließend über eine Amicon-Ultrafiltrationsmembran YM5 entsalzt und schließlich gefriergetrocknet.
Ausbeute: 5,87 g
H₂O-Gehalt (Karl-Fischer): 8,9 %
Gd-Bestimmung (AAS): 15,93 %
T₁-Relaxivität (H2O): 13,22 ± 0,23 l/mmol·sec
(Plasma): 14,39 ± 0,12 l/mmol·sec

### Beispiel 14

### Gd-Komplex des N-(Carboxymethoxyacetyl)-N-(2-hydroxy-propyl-DO3A)-48-Amino-Kaskadenpolymers

1,7 g des in Beispiel 13c beschriebenen Polymers werden in 20 ml H₂O gelöst und durch Zugabe von 1N NaOH auf pH 9 eingestellt. Dazu werden 772 mg (6 mmol) Diglycolsäure-anhydrid (Fluka) portionsweise unter Rühren zugegeben, wobei der pH-Wert durch Zugabe von 2N NaOH auf pH 9 gehalten wird. Nach beendeter Zugabe wird 15 Minuten nachgerührt, mit verd. Salzsäure neutralisiert, ultrafiltriert (Amicon YM5) und schließlich gefriergetrocknet.
Ausbeute: 1,90 g
H₂O-Gehalt (Karl-Fischer): 10,7 %
Gd-Bestimmung (AAS): 14,93 %
T₁-Relaxivitat (H₂O): 13,52 ± 0,22 l/mmol·sec
(Plasma): 15,01 ± 0,37 l/mmol·sec

### Beispiel 15

### Konjugat aus dem Gd-Komplex des 10-(9-Brom-2-hydroxy-8-oxo-4-oxa-7-azanonyl)-1,4,7-triscarboxymethyl-1,4,7,10-tetraazacyclododecans mit dem Gd-Komplex des N-(5-Hydroxy-3-oxahexyl-DO3A)-48-Amino-Kaskadenpolymer

1,7 g des in Beispiel 13c beschriebenen Polymers werden in 20 ml H₂O gelöst und durch Zugabe von 2N NaOH auf pH 9.5 eingestellt. Dazu werden bei 40°C 4,43 g (6 mmol) des in Beispiel 3 beschriebenen Gd-Komplexes unter Rühren zugegeben, wobei der pH-Wert durch Zugabe von 2N NaOH auf pH 9,5 gehalten wird. Nach 24 Stunden bei 40°C wird mit verdünnter Salzsäure neutralisiert, ultrafiltriert (AMICON YM5) und schließlich gefriergetrocknet.
Ausbeute: 2,45 g
H₂O-Gehalt (Karl-Fischer): 9,7 %
Gd-Bestimmung (AAS): 15,72 %
T₁-Relaxivität (H₂O): 13,07 ± 0,23 l/mmol·sec
(Plasma): 14,39 ± 0,15 l/mmol·sec

## Patentansprüche

1. Kaskaden-Polymer-Komplexe der allgemeinen Formel I, die komplexbildende Liganden enthalten, 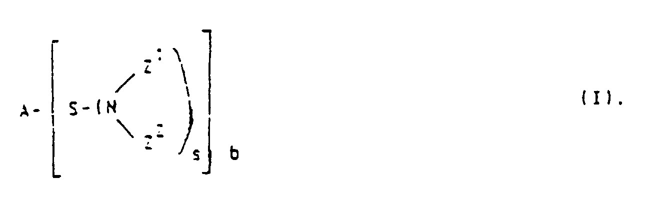 worin
A für einen Stickstoff-haltigen Kaskadenkern der Basismultiplizität b,
S für eine Reproduktionseinheit,
N für ein Stickstoffatom,
Z¹ und Z² für die erste bis vorletzte Generation jeweils für 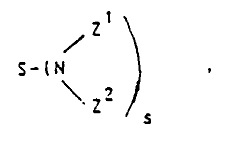 für die letzte Generation dagegen
Z¹ für ein Wasserstoffatom, einen gegebenenfalls 1 bis 3 Carboxyl-, 1 bis 3 Sulfonsäure-, 1 bis 5 Hydroxygruppen und/oder 1 bis 3 Sauerstoffatome enthaltenden C₁-C₁₀-Alkyl-, C₂-C₁₀-Acyl- oder C₁-C₁₀-Alkylsulfonylrest oder für den Rest eines Komplexes K und
Z² für den Rest eines Komplexes K
b für die Ziffern 1 bis 50 und
s für die Ziffern 1 bis 3 stehen,
K einen an die terminalen Stickstoffatome der letzten Generation über V gebundenen Komplex-Rest der allgemeinen Formel (IB) bedeutet 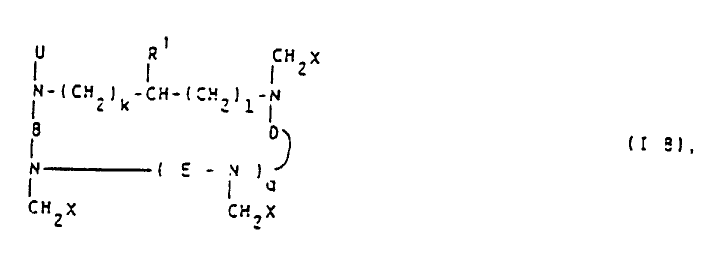 worin
k für die Ziffern 1,2,3,4 oder 5,
l für die Ziffern 0,1,2,3,4 oder 5,
q für die Ziffern 0,1 oder 2,
U für CH₂X oder V
X für den Rest -COOH stehen,
B,D und E die gleich oder verschieden sind, jeweils für die Gruppe -(CH₂)ₐ- mit a in der Bedeutung der Ziffern 2,3,4 oder 5,
R¹ für V oder ein Wasserstoffatom
stehen,
mit der Maßgabe, daß R¹ nur dann für V steht, wenn U gleichzeitig CH₂X bedeutet, und daß U nur dann für V steht, wenn gleichzeitig R¹ ein Wasserstoffatom bedeutet, sowie der Maßgabe, daß gewünschtenfalls ein Teil der COOH-Gruppen als Ester und/oder Amid vorliegt,
V eine gegebenenfalls Imino-, Phenylen-, Phenylenoxy-Phenylenimino-, Amid-, Hydrazid-, Ureido-, Thioureido-, Carbonyl-, Estergruppe(n), Sauerstoff-, Schwefel- und/oder Stickstoff-Atom(e) enthaltende gegebenenfalls durch Hydroxy-, Mercapto-, Imino-, Epoxy-, Oxo-, Thioxo- und/oder Aminogruppen (n) substituierte geradkettige, verzweigte, gesättigte oder ungesättigte C₁-C₂₀-Alkylengruppe bedeutet,
wobei die Reproduktionseinheiten S nur für eine Generation identisch sein müssen und die Anzahl der Generationen 2 bis 10 beträgt, und wobei die Polymeren in den Komplexresten K mindestens fünf Ionen eines Elements der Ordnungszahlen 21 bis 29, 39, 42, 44 oder 57 bis 83 komplexiert sowie gegebenenfalls Kationen anorganischer und/oder organischer Basen, Aminosäuren oder Aminosäureamide enthalten, zur Verwendung als Kontrastmittel für die NMR- oder Röntgen-Diagnostik.

2. Kaskaden-Polymer-Komplexe gemäß Anspruch 1, dadurch gekennzeichnet, daß S
-(CH₂)₂-CONH-(CH₂)ₐ-
oder 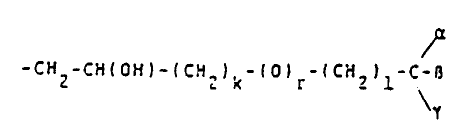 bedeutet,
worin
α und β jeweils für ein Wasserstoffatom oder (CH₂)ₒ,
γ für (CH₂)_{f},
f für die Ziffern 1, 2, 3, 4 oder 5,
o für die Ziffern 0, 1, 2, 3, 4 oder 5, wobei l und o nicht gleichzeitig für die Ziffer 0 stehen sollen, und
r für die Ziffern 0 oder 1
stehen und
a, k und l die oben angegebene Bedeutung haben.

3. Kaskaden-Polymer-Komplex gemäß Anspruch 1, dadurch gekennzeichnet, daß A ein Stickstoffatom,
NR²R³R⁴, 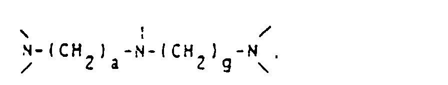 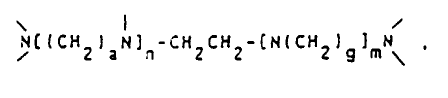 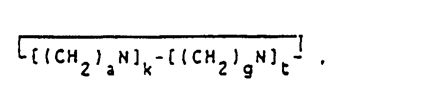 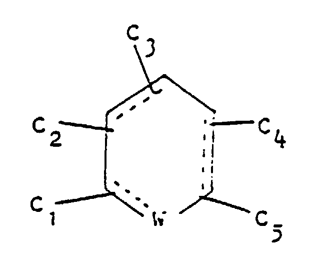 oder 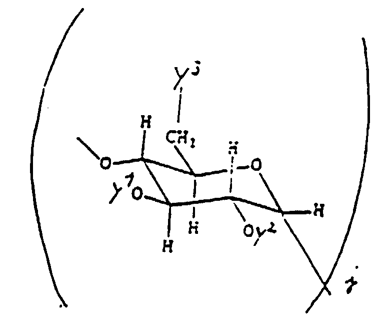 bedeutet, worin
R², R³ und R⁴ jeweils unabhängig voneinander für eine kovalente Bindung oder (CH₂)ₖ-(C₆H₄)ᵣ-(CH₂)-N〈 ,
g für die Ziffern 2. 3, 4 oder 5,
t für die Ziffern 1, 2, 3, 4, 5, 6, 7 oder 8,
W für CH, CH₂, NH oder ein Stickstoffatom,
C₁ für (CH₂)ₖ-N〈 ,
C₂, C₃, C₄ und C₅ jeweils unabhängig für ein Wasserstoffatom oder (CH₂)_{f}-N〈 ,
j für die Ziffern 6, 7 oder 8,
Y¹ und Y² jeweils unabhängig voneinander für ein Wasserstoffatom, CH₂-CH(OH)-CH₂N〈 oder (CH₂)ₐ-N〈 und
Y³ für ein Stickstoffatom, O-CH₂-CH(OH)-CH₂N〈 oder O-(CH₂)_{g}-N〈
--- für eine Einfach- oder Doppelbindung
stehen, mit der Maßgabe, daß wenn Y³ für ein Stickstoffatom steht, Y¹ und Y² für Wasserstoff stehen.

4. Kaskaden-Polymer-Komplex gemäß Anspruch 1, dadurch gekennzeichnet, daß V für eine
-CH₂-CH(OH)-CH₂-O-CH₂CH₂-;
-CH₂-CH(OH)-CH₂-; 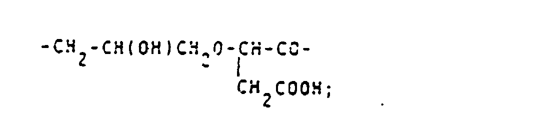 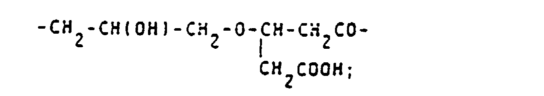 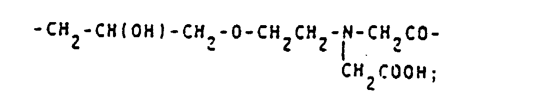 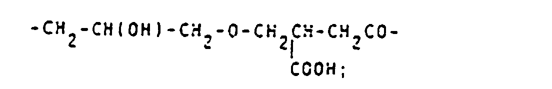 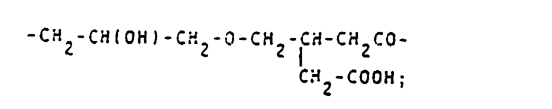 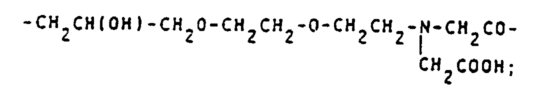 -CH₂-CH(OH)-CH₂-OCH₂-C₆H₄-NH-CO-CH₂-;
-CH₂-CH(OH)-CH₂-OCH₂-C₆H₄-NH-CO-CH₂-S-(CH₂)₄-S-; 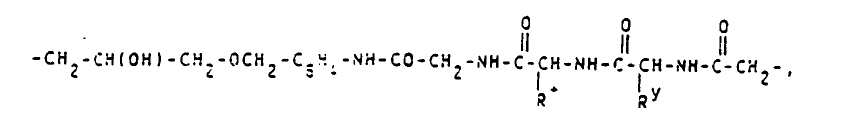 worin R⁺ und R^{y} für natürliche Aminosäurereste stehen;
-CH₂-CH(OH)-CH₂-O-(CH₂)₂-NHCS-;
-CH₂-CH(OH)-CH₂-NHCS-;
-CH₂-CH(OH)-CH₂-O-(CH₂)₂-O-(CH₂)₂NHCS-;
-CH₂-CH(OH)-CH₂-O-(CH₂)₂-NH-CO-CH₂-;
-CH₂-CH(OH)-CH₂-O-C₆H₄-NHCS-;
-CH₂-CH(OH)-CH₂-O-C₆H₄-NHCO-;
-CH₂-CH(OH)-CH₂-O-CH₂-C₆H₄-NHCS-;
-CH₂-O-C₆H₄-CH₂-; -CH₂-CH(OH)-CH₂-O-C₆H₄-CH₂-; -C(= NH)-O-C₆H₄-CH₂-;
-(CH₂)₄-NH-CO-CH₂-O-C₆H₄-CH₂-; -(CH₂)₄-NH-CH₂-CH(OH)-CH₂-O-C₆H₄-CH₂-;
-(CH₂)₃-O-C₆H₄-CH₂-; -CH₂-CO-NH-(CH₂)₃-O-CH₂-; -CH₂-CO-NH-NH-;
-CH₂-CONH-(CH₂)₂-; -CH₂-CO-NH-(CH₂)₁₀-; -CH₂-CONH-(CH₂)₂-S-;
-(CH₂)₄-NH-CO-(CH₂)₈-; -CH₂-CO-NH-(CH₂)₃-NH-; -(CH₂)₃-NH-Gruppe steht.

5. Pharmazeutische Mittel enthaltend mindestens einen Kaskaden-Polymer Komplex nach Anspruch 1, gegebenenfalls mit den in der Galenik üblichen Zusätzen.

6. Verwendung von mindestens einem Kaskaden-Polymer-Komplex gemäß Anspruch 1 für die Herstellung von Mitteln für die NMR- oder Röntgen-Diagnostik.

7. Verfahren zur Herstellung der pharmazeutischen Mittel gemäß Anspruch 5, dadurch gekennzeichnet, daß man den in Wasser oder physiologischer Salzlösung gelösten oder suspendierten Polymer-Komplex, gegebenenfalls mit den in der Galenik üblichen Zusätzen, in eine für die enterale oder parenterale Applikation geeignete Form bringt.

## Claims

1. Cascade polymer complexes of the general formula I containing complex-forming ligands 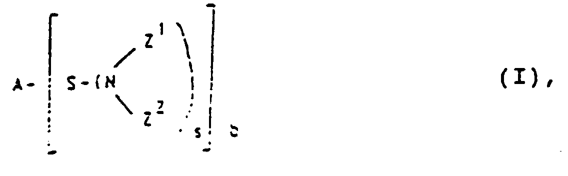 wherein
A represents a nitrogen-containing cascade nucleus of base multiplicity b,
S represents a reproduction unit,
N represents a nitrogen atom,
Z¹ and Z² for the first to penultimate generation each represents 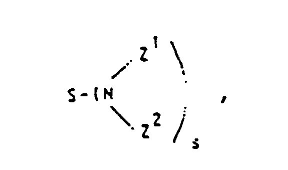 but for the last generation
Z¹ represents a hydrogen atom, a C₁-C₁₀-alkyl, C₂-C₁₀-acyl or C₁-C₁₀-alkylsulphonyl radical optionally containing from 1 to 3 carboxy groups, from 1 to 3 sulphonic acid groups, from 1 to 5 hydroxy groups and/or from 1 to 3 oxygen atoms, or represents the radical of a complex K and
Z² represents the radical of a complex K
b represents the numbers 1 to 50 and
s represents the numbers 1 to 3,
K represents a complex radical bonded to the terminal nitrogen atoms of the last generation via V of the general formula (IB) 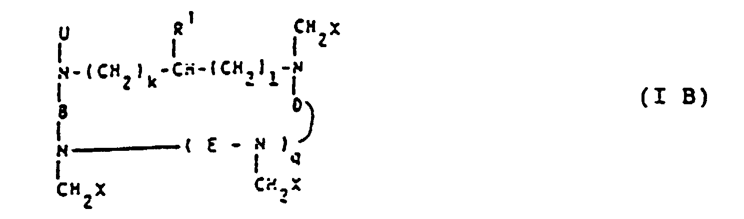 wherein
k represents the numbers 1, 2, 3, 4 or 5,
l represents the numbers 0, 1, 2, 3, 4 or 5,
q represents the numbers 0, 1 or 2,
U represents CH₂X or V,
X represents the radical -COOH
B, D and E, which are identical or different, each represents the group -(CH₂)ₐ- wherein a represents the numbers 2, 3, 4 or 5,
R¹ represents V or a hydrogen atom,
with the proviso that R¹ represents V only when U at the same time represents CH₂X, and that U represents V only when R¹ at the same time represents a hydrogen atom, and with the proviso that, if desired, some of the COOH groups are in the form of esters and/or amides,
V representing a straight-chain, branched, saturated or unsaturated C₁-C₂₀-alkylene group optionally containing imino; phenylene, phenyleneoxy, phenyleneimino, amide, hydrazide, ureido, thioureido, carbonyl, ester group(s), oxygen, sulphur and/or nitrogen atom(s), and optionally substituted by hydroxy, mercapto, imino, epoxy, oxo, thioxo and/or by amino group(s),
wherein the reproduction units S have to be identical only for one generation and the number of generations is from 2 to 10, and wherein the polymers in the complex radicals K contain complexed at least five ions of an element of atomic number 21 to 29, 39, 42, 44 or 57 to 83 and optionally cations of inorganic and/or organic bases, amino acids or amino acid amides, for use as contrast media for NMR and X-ray diagnostics.

2. Cascade polymer complexes according to claim 1, characterised in that S represents
-(CH₂)₂-CONH-(CH₂)ₐ-
or 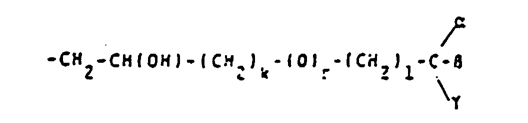 wherein
α and β each represents a hydrogen atom or (CH₂)ₒ,
γ represents (CH₂)_{f},
f represents the numbers 1, 2, 3, 4 or 5,
o represents the numbers 0, 1, 2, 3, 4 or 5, wherein I and o should not represent the number 0 at the same time, and
r represents the number 0 or 1
and
a, k and I have the meanings given above.

3. Cascade polymer complex according to claim 1, characterised in that A represents a nitrogen atom,
NR²R³,R⁴, 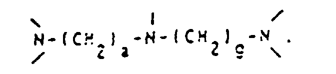 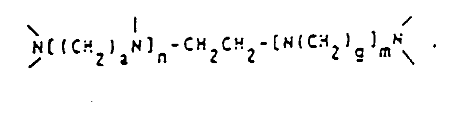 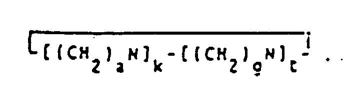 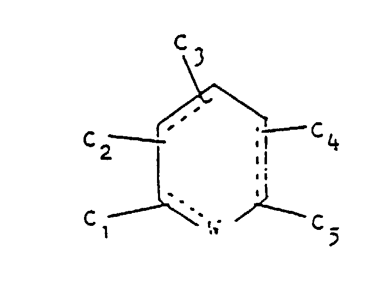 or 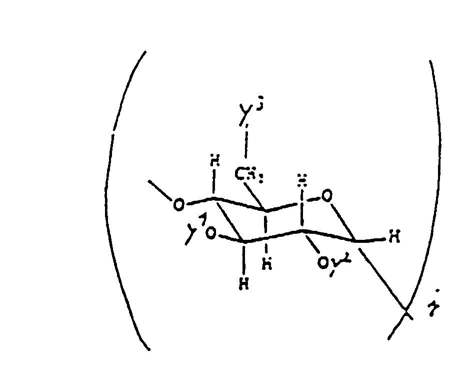 wherein
R², R³ and R⁴ each independently of the others represents a covalent bond or (CH₂)ₖ-(C₆H₄)ᵣ-(CH₂)ₗ-N〈 ,
g represents the numbers 2, 3, 4 or 5,
t represents the numbers 1, 2, 3, 4, 5, 6, 7 or 8,
W represents CH, CH₂, NH or a nitrogen atom,
C₁ represents (CH₂)ₖ-N〈 ,
C₂, C₃, C₄ and C₅ each independently represents a hydrogen atom or (CH₂)_{f}-N〈 ,
j represents the numbers 6, 7 or 8,
Y¹ and Y² each independently of the other represents a hydrogen atom, CH₂-CH(OH)-CH₂N〈 or (CH₂)ₐ-N〈 ,
Y³ represents a nitrogen atom, O-CH₂-CH(OH)-CH₂N〈 or O-(CH₂)_{g}-N〈 , and
--- represents a single or double bond,
with the proviso that, when Y³ is a nitrogen atom, Y¹ and Y² represent hydrogen.

4. Cascade polymer complex according to claim 1, characterised in that V represents a
-CH₂-CH(OH)-CH₂-O-CH₂CH₂-;
-CH₂CH(OH)-CH₂-; 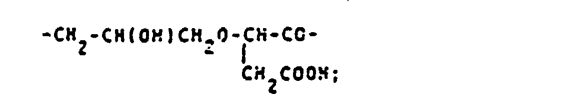 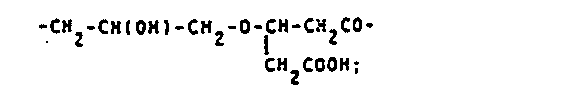 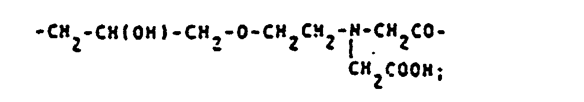 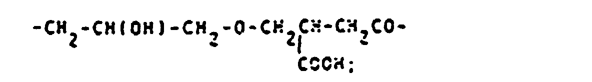 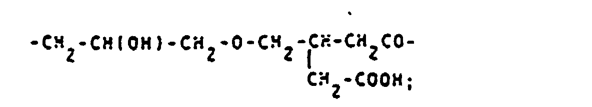 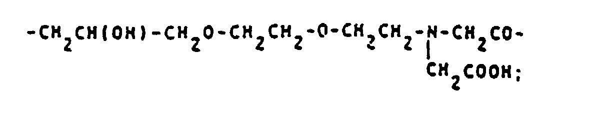 -CH₂-CH(OH)-CH₂-OCH₂-C₆H₄-NH-CO-CH₂-;
-CH₂-CH(OH)-CH₂-OCH₂-C₆H₄-NH-CO-CH₂-S-(CH₂)₄-S-; 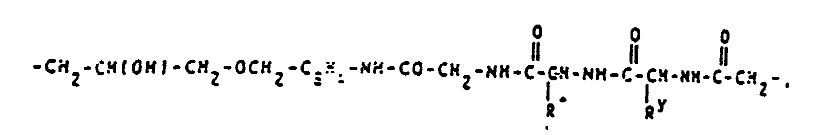 wherein R⁺ and R^{y} represent natural amino acids;
-CH₂-CH(OH)-CH₂-O-(CH₂)₂-NHCS-;
-CH₂-CH(OH)-CH₂-NHCS-;
-CH₂-CH(OH)-CH₂-O-(CH₂)₂-O-(CH₂)₂NHCS-;
-CH₂CH(OH)-CH₂-O-(CH₂)₂-NH-CO-CH₂-;
-CH₂-CH(OH)-CH₂-O-C₆H₄-NHCS-;
-CH₂-CH(OH)-CH₂-O-C₆H₄-NHCO-;
-CH₂-CH(OH)-CH₂-O-CH₂-C₆H₄-NHCS-;
-CH₂-O-C₆H₄-CH₂-; -CH₂-CH(OH)-CH₂-O-C₆H₄-CH₂-; -C(= NH)-O-C₆H₄-CH₂-;
-(CH₂)₄-NH-CO-CH₂-O-C₆H₄-CH₂-;-(CH₂)₄-NH-CH₂-CH(OH)-CH₂-O-C₆H₄-CH₂-;
-(CH₂)₃-O-C₆H₄-CH₂-; -CH₂-CO-NH-(CH₂)₃-O-CH₂-; -CH₂-CO-NH-NH-;
-CH₂-CONH-(CH₂)₂-; -CH₂-CO-NH-(CH₂)₁₀-; -CH₂-CONH-(CH₂)₂-S-;
-(CH₂)₄-NH-CO-(CH₂)₈-; -CH₂-CO-NH-(CH₂)₃-NH-; -(CH₂)₃-NH-group.

5. Pharmaceutical agents containing at least one cascade polymer complex according to claim 1, optionally together with the additives customary in galenical pharmacy.

6. Use of at least one cascade polymer complex according to claim 1 for the manufacture of media for NMR and X-ray diagnostics.

7. Process for the manufacture of the pharmaceutical agents according to claim 5, characterised in that the polymer complex, dissolved or suspended in water or physiological saline solution, is brought into a form suitable for enteral or parenteral administration, optionally together with the additives customary in galenical pharmacy.

## Revendications

1. Complexes de polymère en cascade de la formule générale I, contenant des ligands complexants 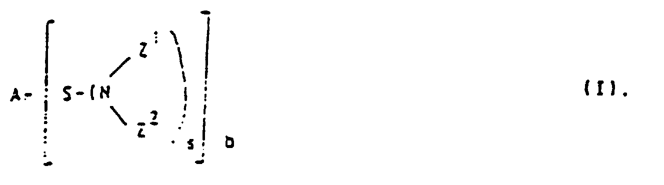 dans laquelle
A représente un noyau de cascade contenant de l'azote présentant une multiplicité de base b,
S représente une unité de reproduction,
N représente un atome d'azote,
Z¹ et Z² représentent, pour la première à l'avant-dernière génération, 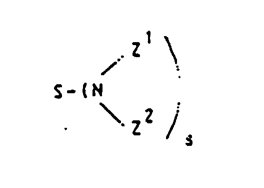 tandis que pour la dernière génération
Z¹ représente un atome d'hydrogène, un radical alkyle en C₁-C₁₀, acyle en C₂-C₁₀ ou alkylsulfonyle en C₁-C₁₀ contenant éventuellement 1 à 3 groupes carboxyle, 1 à 3 groupes d'acide sulfonique, 1 à 5 groupes hydroxy et/ou 1 à 3 atomes d'oxygène ou le radical d'un complexe K, et
Z² représente le radical d'un complexe K
b représente les chiffres 1 à 50, et
s représente les chiffres 1 à 3,
K représente un radical de complexe lié aux atomes d'azote terminaux de la dernière génération par l'intermédiaire de V répondant à la formule générale (IB) 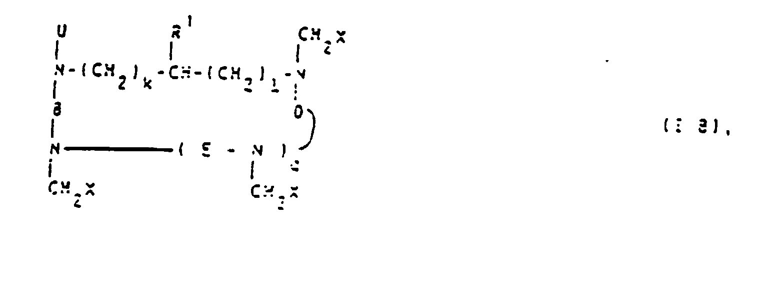 où
k représente les chiffres 1, 2, 3, 4 ou 5,
l représente les chiffres 0, 1, 2, 3, 4 ou 5,
q représente les chiffres 0, 1 ou 2,
U représente CH₂X ou V,
X représente le radical -COOH
B, D et E, qui sont identiques ou différents, représentent chacun le groupe -(CH₂)ₐ-, où a représente les chiffres 2, 3, 4 ou 5,
R¹ représente V ou un atome d'hydrogène,
à la condition que R¹ représente uniquement V lorsque U représente simultanément CH₂X, et que U représente uniquement V, lorsque simultanément R¹ représente un atome d'hydrogène, ainsi qu'à la condition qu'éventuellement une partie des groupes COOH se présente sous la forme d'esters et/ou d'amides,
V représentant un groupe alkylène en C₁-C₂₀ saturé ou insaturé, linéaire ou ramifié, éventuellement substitué par un ou des groupes hydroxy, mercapto, imino, époxy, oxo, thioxo et/ou amino et contenant éventuellement un ou des groupes imino, phénylène, phénylèneoxy, phénylèneimino, amide, hydrazide, uréido, thiouréido, carbonyle, ester, et un ou des atomes d'oxygène, de soufre et/ou d'azote,
les unités de reproduction S ne devant être identiques que pour une génération et le nombre des générations étant de 2 à 10, et les polymères dans les radicaux de complexe K contiennent compléxes au moins cinq ions d'un élément des nombres atomiques 21 à 29, 39, 42, 44 ou 57 à 83 ainsi qu'éventuellement des cations de bases inorganiques et/ou organiques, d'aminoacides ou d'amides d'aminoacides, pour l'utilisation en produits de contraste de diagnostic de RMN ou aux rayons X.

2. Complexes de polymère en cascade suivant la revendication 1, caractérisés en ce que S représente -(CH₂)₂-CONH-(CH₂)ₐ- ou 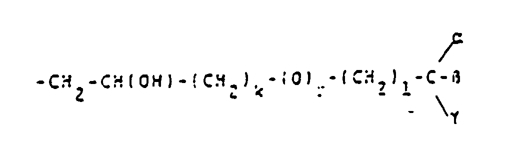 où
α et β représentent chacun un atome d'hydrogène ou (CH₂)ₒ,
γ représente (CH₂)_{f},
f représente les chiffres 1, 2, 3, 4 ou 5,
o représente les chiffres 0, 1, 2, 3, 4 ou 5, l et o ne pouvant pas représenter simultanément le chiffre 0, et
r représente les chiffres 0 ou 1,
a, k et l ayant la signification indiquée ci-dessus.

3. Complexe de polymère en cascade suivant la revendication 1, caractérisés en ce que A représente un atome d'azote, NR²R³R⁴, 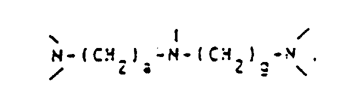 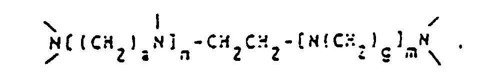 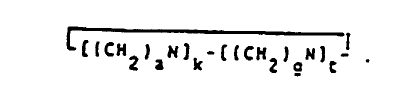 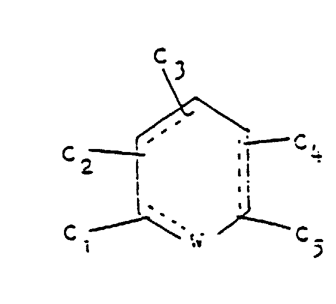 ou 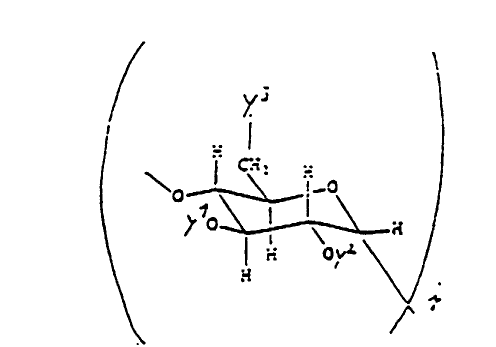 où
R², R³ et R⁴ représentent indépendamment l'un de l'autre une liaison covalente ou (CH₂)ₖ-(C₆H₄)ᵣ(CH₂)ₗ-N〈 ,
g représente les chiffres 2, 3, 4 ou 5,
t représente les chiffres 1, 2, 3, 4, 5, 6, 7 ou 8,
W représente CH, CH₂, NH ou un atome d'azote,
C₁ représente (CH₂)ₖ-N〈 ,
C₂, C₃, C₄ et C₅ représentent chacun indépendamment un atome d'hydrogène ou (CH₂)_{f}-N〈 ,
j représente les chiffres 6, 7 ou 8,
Y¹ et Y² représentent chacun indépendamment l'un de l'autre un atome d'hydrogène, CH₂-CH(OH)-CH₂N〈 ou (CH₂)ₐ-N〈 et
Y³ représente un atome d'azote, O-CH₂-CH(OH)-CH₂N〈 ou O-(CH₂)_{g}-N〈
--- représente une simple ou double liaison,
à la condition que, lorsque Y³ représente un atome d'azote, Y¹ et Y² représentent de l'hydrogène.

4. Complexe de polymère en cascade suivant la revendication 1, caractérisés en ce que V représente un groupe
-CH₂-CH(OH)-CH₂-O-CH₂CH₂-;
-CH₂-CH(OH)-CH₂-; 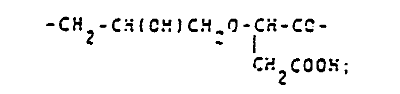 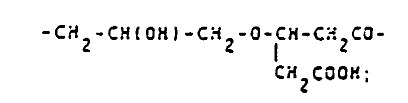 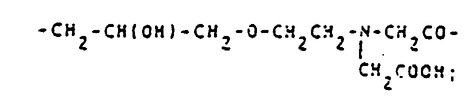 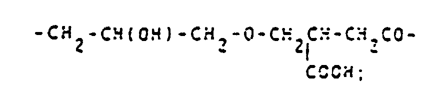 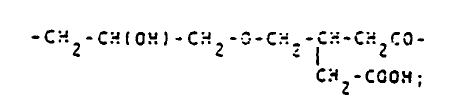 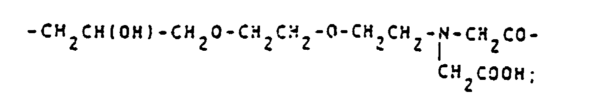 -CH₂-CH(OH)-CH₂-OCH₂-C₆H₄-NH-CO-CH₂-;
-CH₂-CH(OH)-CH₂-OCH₂-C₆H₄-NH-CO-CH₂-S-(CH₂)₄-S-; 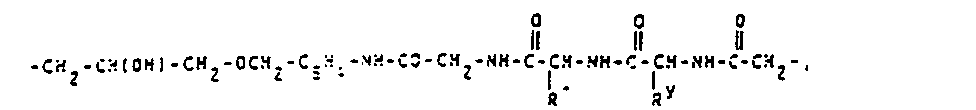 où R⁺ et R^{y} sont des radicaux d' aminoacides naturels,
-CH₂-CH(OH)-CH₂-O-(CH₂)₂-NHCS-;
-CH₂-CH(OH)-CH₂-NHCS-;
-CH₂-CH(OH)-CH₂-O-(CH₂)₂-O-(CH₂)₂NHCS-;
-CH₂-CH(OH)-CH₂-O-(CH₂)₂-NH-CO-CH₂-;
-CH₂-CH(OH)-CH₂-O-C₆H₄-NHCS-;
-CH₂-CH(OH)-CH₂-O-C₆H₄-NHCO-;
-CH₂-CH(OH)-CH₂-O-CH₂-C₆H₄-NHCS-;
-CH₂-O-C₆H₄-CH₂-; -CH₂-CH(OH)-CH₂-O-C₆H₄-CH₂-; -C(= NH)-O-C₆H₄-CH₂-;
-(CH₂)₄-NH-CO-CH₂-O-C₆H₄-CH₂-; -(CH₂)₄-NH-CH₂-CH(OH)-CH₂-O-C₆H₄-CH₂-;
-(CH₂)₃-O-C₆H₄-CH₂-; -CH₂-CO-NH-(CH₂)₃-O-CH₂-; -CH₂-CO-NH-NH-;
-(CH₂)-CONH-(CH₂)₂-; -CH₂-CO-NH-(CH₂)₁₀-; -CH₂-CONH-(CH₂)₂-S-;
-(CH₂)₄-NH-CO-(CH₂)₈-; -CH₂-CO-NH-(CH₂)₃-NH-; -(CH₂)₃-NH-.

5. Produits pharmaceutiques contenant au moins un complexe de polymère en cascade suivant la revendication 1, éventuellement avec les additifs courants en pharmacie galénique.

6. Utilisation d'au moins un complexe de polymère en cascade suivant la revendication 1 pour la fabrication de produits de diagnostic de RMN ou aux rayons X.

7. Procédé de préparation des produits pharmaceutiques suivant la revendication 5, caractérisé en ce qu'on amène le complexe de polymère dissous ou en suspension dans l'eau ou une solution saline physiologique, éventuellement avec les additifs courants en pharmacie galénique, sous une forme appropriée pour l'application entérale ou parentérale.
